# EUROPEAN PATENT APPLICATION

(11) **EP 4 289 862 A1**
(43) Date of publication of application: **13.12.2023**
(21) Application number: 21900066.8
(22) Date of filing: 02.12.2021
(51) Int. Cl.: C07K 16/28, A61K 39/395, C12N 15/13, A61P 35/00

(54) **ANTI-HUMAN B7-H3 ANTIBODY AND APPLICATION THEREOF**

(30) Priority: 02.12.2020 CN 202011398727
(71) Applicant: Mabwell (Shanghai) Bioscience Co., Ltd., Shanghai 201210 (CN)
(72) Inventor: JIAO, Shasha, Shanghai 201210 (CN); WANG, Rongjuan, Shanghai 201210 (CN); WANG, Shuang, Shanghai 201210 (CN); ZHANG, Jiao, Shanghai 201210 (CN); ZHANG, Chang, Shanghai 201210 (CN); ZENG, Dadi, Shanghai 201210 (CN)
(74) Representative: Schiweck Weinzierl Koch Patentanwälte Partnerschaft mbB
(86) International application number: PCT/CN2021/135091
(87) International publication number: WO 2022/117040

(57) **Abstract**

Provided are an anti-human B7-H3 monoclonal antibody and an application thereof. By using a recombinant human B7-H3 extracellular region as an immunogen, a murine anti-human B7-H3 monoclonal antibody can be prepared by means of a hybridoma technology, and a murine anti-human B7-H3 antibody can bind to various domains of the B7-H3 extracellular region. A human-mouse chimeric antibody constructed on the basis of a murine antibody can specifically bind to B7-H3 of a cell surface. A humanized antibody prepared by means of CDRs transplantation and CDRs region mutation retains the ability to specifically bind to the human B7-H3 extracellular region and cell membrane surface B7-H3, and can be mediated for internalization by the cell membrane surface B7-H3.

## Description

### PRIORITY INFORMATION

The present patent application claims priority to and the benefit of Chinese patent application No. 202011398727.1 filed with the China National Intellectual Property Administration on December 2, 2020, which is incorporated herein by reference in its entirety.

### TECHNICAL FIELD

The invention belongs to the field of antibody engineering, and specifically is directed to an anti-human B7-H3 monoclonal antibody and applications thereof. In particular, the invention is directed to a monoclonal antibody, a chimeric antibody, and a humanized antibody having a high affinity for human B7-H3, having cross reaction with monkey B7-H3, and not binding to murine B7-H3, as well as applications thereof.

### BACKGROUND OF THE INVENTION

B7-H3, also known as CD276, is a type I transmembrane protein belonging to the B7-CD28 superfamily. The molecules of the B7 family can provide stimulatory signals to enhance and maintain T cell immune responses, and can also produce inhibitory signals to limit and attenuate T cell immune reactions. Human B7-H3 protein is present in two forms: membrane-bound and soluble forms. The membrane-bound B7-H3 protein is mainly distributed on the surface of tumor cells, and also is found in nuclei, transporters, exosomes, and the like in the cells. The soluble B7-H3 is hydrolyzed from the cell membrane by proteases, and high levels of soluble B7-H3 can be detected in sera of tumor patients, suggesting that B7-H3 can be used as a biomarker (xiec, 2016). The recent clinical and commercial successes of anti-cancer antibodies have fostered a great interest in antibody-based therapeutics. Therefore, antibody therapeutics based on B7-H3 for the treatment of cancer remain to be researched.

### SUMMARY OF THE INVENTION

To address the defects of insufficient affinity and specificity of anti-human B7-H3 antibodies in the prior arts for the B7-H3 protein on the membrane surface which result in off-target effects and adverse reactions, the present disclosure is to provide an anti-human B7-H3 monoclonal antibody and applications thereof. Murine anti-human B7-H3 monoclonal antibodies were prepared by hybridoma technology with the recombinant human B7-H3 extracellular domain as an immunogen. The murine anti-human B7-H3 antibodies were capable of specifically binding to various B7-H3 extracellular domains, and human-murine chimeric antibodies constructed based on the murine antibodies were capable of specifically binding to B7-H3 on the cell surface. Humanized antibodies prepared through CDR grafting and CDR mutation retained the ability to specifically bind to human B7-H3 extracellular domain and B7-H3 on cell membrane surface, and also could internalize through the mediation by B7-H3 on cell membrane surface. Anti-human B7-H3 humanized antibody-drug conjugates (ADCs) were shown to be capable of specifically killing tumor cells in a cytologic experiment and a nude-mouse transplanted tumor model. The anti-human B7-H3 antibodies had affinities less than 5E-10 M for human B7-H3 extracellular domain, were capable of specifically binding to B7-H3 on the surfaces of recombinant cells and tumor cells, and contributed to remarkable tumor killing effects in cells and animal models, and also no obvious adverse reactions were observed. Therefore, the anti-human B7-H3 antibodies possess a good clinical antitumor application prospect.

### Antibody and preparation method therefor

According to one aspect of the embodiments of the invention, the present disclosure provides an anti-human B7-H3 antibody or fragment thereof, comprising a heavy chain variable region and a light chain variable region. According to the embodiments of the invention, the heavy chain variable region (VH) comprises complementarity-determining regions (CDRs) 1, 2 and 3, wherein the VH CDR1 comprises an amino acid sequence having at least 75% identity to the amino acid sequence of a selected VH CDR1, the VH CDR2 comprises an amino acid sequence having at least 75% identity to the amino acid sequence of a selected VH CDR2, and the VH CDR3 comprises an amino acid sequence having at least 75% identity to the amino acid sequence of a selected VH CDR3; and, the light chain variable region (VL) comprises CDRs 1, 2 and 3, wherein the VL CDR1 comprises an amino acid sequence having at least 75% identity to the amino acid sequence of a selected VL CDR1, the VL CDR2 comprises an amino acid sequence having at least 75% identity to the amino acid sequence of a selected VL CDR2, and the VL CDR3 comprises an amino acid sequence having at least 75% identity to the amino acid sequence of a selected VL CDR3; wherein the amino acid sequences of the selected VH CDRs 1, 2 and 3 and the amino acid sequences of the selected VL CDRs 1, 2 and 3 are selected from the group consisting of:
the amino acid sequences of the selected VH CDRs 1, 2 and 3 are as shown in SEQ ID NOs: 40, 41 and 42 respectively, and the amino acid sequences of the selected VL CDRs 1, 2 and 3 are as shown in SEQ ID NOs: 43, 44 and 45 respectively;
the amino acid sequences of the selected VH CDRs 1, 2 and 3 are as shown in SEQ ID NOs: 46, 47 and 48 respectively, and the amino acid sequences of the selected VL CDRs 1, 2 and 3 are as shown in SEQ ID NOs: 49, 50 and 51 respectively; and
the amino acid sequences of the selected VH CDRs 1, 2 and 3 are as shown in SEQ ID NOs: 52, 53 and 54 respectively, and the amino acid sequences of the selected VL CDRs 1, 2 and 3 are as shown in SEQ ID NOs: 55, 56 and 57 respectively.

The anti-human B7-H3 antibody or fragment thereof according to the embodiments of the invention are capable of specifically binding to sites in human B7-H3 extracellular domain and B7-H3 on cell membrane surface, and has a strong affinity, e.g., an affinity less than 5E-10 M in some embodiments; and in addition, has a good tumor inhibiting effect and a low toxicity, thereby possessing a good clinical application prospect.

Further, the antibody or the antigen-binding fragment according to the above embodiments of the invention may have the additional technical features as follows:
according to the embodiments of the invention, the amino acid sequence having at least 75% identity to the selected VL CDR3 which comprises the amino acid sequence as shown in SEQ ID NO: 51 is QQWSX₁X₂PLT, wherein X₁ is S or A, and X₂ is N, A, S, or Q.

According to the embodiments of the invention, the VH comprises an amino acid sequence having at least 75% identity to SEQ ID NO: 1, and the VL comprises an amino acid sequence having at least 75% identity to SEQ ID NO: 2.

According to the embodiments of the invention, the VH comprises an amino acid sequence having at least 75% identity to SEQ ID NO: 3, and the VL comprises an amino acid sequence having at least 75% identity to SEQ ID NO: 4.

According to the embodiments of the invention, the VH comprises an amino acid sequence having at least 75% identity to SEQ ID NO: 5, and the VL comprises an amino acid sequence having at least 75% identity to SEQ ID NO: 6.

Specifically, the at least 75% identity can be at least 82%, preferably at least 85%, more preferably at least 90%, further preferably at least 91%, 92%, 93%, 94%, 95%, 96%, 97%, 98%, or even 99% identity or any percentage of identity greater than or equal to 75%. More specifically, 0, 1, 2, or more amino acids may be inserted, deleted or substituted.

It is further explained that the sequence of the heavy chain variable region of the antibody or the antigen-binding fragment can be obtained by inserting one or more amino acids to, deleting one or more amino acids from, mutating one or more amino acids in, or modifying one or more amino acids in the amino acid sequence as shown in SEQ ID NO: 1, SEQ ID NO: 2, SEQ ID NO: 3, SEQ ID NO: 4, SEQ ID NO: 5, or SEQ ID NO:6.

According to the embodiments of the invention, the VH comprises the amino acid sequence as shown in SEQ ID NO: 11 or 17, and the VL comprises the amino acid sequence as shown in SEQ ID NO: 12, 13, 14, 15, or 16.

According to the embodiments of the invention, the VH comprises the amino acid sequence as shown in SEQ ID NO: 18, 21, 22, or 23, and the VL comprises the amino acid sequence as shown in SEQ ID NO: 19, 20, 58, 59, or 60.

According to the embodiments of the invention, the VH comprises the amino acid sequence as shown in SEQ ID NO: 24, 34, 35, 36, or 37, and the VL comprises the amino acid sequence as shown in SEQ ID NO: 25, 26, 27, 28, 29, 30, 31, 32, or 33.

According to the embodiments of the invention, the anti-human B7-H3 antibody or fragment thereof are capable of specifically binding to human B7-H3 extracellular domain, and has an affinity constant (KD) less than 5E-10 M for the human B7-H3 extracellular domain.

According to the embodiments of the invention, the anti-human B7-H3 antibody or fragment thereof has cross reaction with monkey B7-H3, and has no binding activity to murine B7-H3.

According to the embodiments of the invention, the anti-human B7-H3 antibody or fragment thereof has weak cross reaction with monkey B7-H3, and its binding activity to monkey B7-H3 is lower than its binding activity to human B7-H3.

According to the embodiments of the invention, the anti-human B7-H3 antibody or fragment thereof are capable of specifically binding to B7-H3 on the cell surface and internalizing into the cell through the mediation by B7-H3.

Further, according to another aspect of the invention, the present disclosure provides an anti-human B7-H3 antibody or fragment thereof. According to the embodiments of the invention, the anti-human B7-H3 antibody or fragment thereof are capable of specifically binding to human B7-H3 extracellular domain, and has an affinity constant (KD) less than 5E-10 M for the human B7-H3 extracellular domain.

According to the embodiments of the invention, the anti-human B7-H3 antibody or fragment thereof has cross reaction with monkey B7-H3, and has no binding activity to murine B7-H3.

According to the embodiments of the invention, the anti-human B7-H3 antibody or fragment thereof has weak cross reaction with monkey B7-H3, and its binding activity to monkey B7-H3 is lower than its binding activity to human B7-H3.

According to the embodiments of the invention, the anti-human B7-H3 antibody or fragment thereof are capable of specifically binding to B7-H3 on the cell surface and internalizing into the cell through the mediation by B7-H3.

According to the embodiments of the invention, the antibody includes a murine antibody, a rabbit antibody, a human antibody, and a chimeric antibody.

According to the embodiments of the invention, the anti-human B7-H3 antibody or fragment thereof has heavy chain CDRs of the heavy chain variable region as shown by any one amino acid sequence selected from the group consisting of SEQ ID NOs: 1, 3, and 5; and/or has light chain CDRs of the light chain variable region as shown by any one amino acid sequence selected from the group consisting of SEQ ID NO: 2, 4, and 6.

According to the embodiments of the invention, the anti-human B7-H3 antibody or fragment thereof has a heavy chain variable region as shown by any one amino acid sequence selected from the group consisting of SEQ ID NOs: 1, 3, and 5; and/or has a light chain variable region as shown by any one amino acid sequence selected from the group consisting of SEQ ID NO: 2, 4, and 6.

According to the embodiments of the invention, the anti-human B7-H3 antibody or fragment thereof has:
the heavy chain variable region as shown in SEQ ID NO: 1 and the light chain variable region as shown in SEQ ID NO: 2;
the heavy chain variable region as shown in SEQ ID NO: 3 and the light chain variable region as shown in SEQ ID NO: 4; or
the heavy chain variable region as shown in SEQ ID NO: 5 and the light chain variable region as shown in SEQ ID NO: 6.

According to still another aspect of the invention, the present disclosure provides an anti-human B7-H3 humanized antibody or fragment thereof. According to the embodiments of the invention, the anti-human B7-H3 humanized antibody or fragment thereof is obtained through CDR grafting on the basis of the anti-human B7-H3 antibody or fragment thereof described above.

According to the embodiments of the invention, the anti-human B7-H3 humanized antibody or fragment thereof has:
the heavy chain variable region as shown by any one sequence selected from the group consisting of SEQ ID NOs: 11 and 17, or the heavy chain CDRs 1, 2, and 3 thereof;
the light chain variable region as shown by any one sequence selected from the group consisting of SEQ ID NOs: 12, 13, 14, 15, and 16, or the light chain CDRs 1, 2, and 3 thereof.

According to the embodiments of the invention, the anti-human B7-H3 humanized antibody or fragment thereof has:
the heavy chain variable region as shown by any one sequence selected from the group consisting of SEQ ID NOs: 18, 21, 22, and 23, or the heavy chain CDRs 1, 2, and 3 thereof;
the light chain variable region as shown by any one sequence selected from the group consisting of SEQ ID NOs: 19 and 20, or the light chain CDRs 1, 2, and 3 thereof.

According to the embodiments of the invention, the anti-human B7-H3 humanized antibody or fragment thereof has:
the heavy chain variable region as shown by any one sequence selected from the group consisting of SEQ ID NOs: 24, 34, 35, 36, and 37, or the heavy chain CDRs 1, 2, and 3 thereof;
the light chain variable region as shown by any one sequence selected from the group consisting of SEQ ID NOs: 25, 26, 27, 28, 29, 30, 31, 32, and 33, or the light chain CDRs 1, 2, and 3 thereof.

According to still another aspect of the invention, the present disclosure provides a method for preparing an anti-human B7-H3 humanized antibody or fragment thereof. According to the embodiments of the invention, on the basis of a non-human anti-B7-H3 antibody or fragment thereof, CDR grafting technology is utilized to achieve the humanization of heavy chain variable region and/or the humanization of light chain variable region, in order to obtain the anti-human B7-H3 antibody or fragment thereof described above.

In the method for preparing an anti-human B7-H3 humanized antibody or fragment thereof according to the embodiments of the invention, a non-human monoclonal antibody is engineered for humanization using genetic cloning, DNA recombination technology and the like; and after the engineering, the antibody expressed and binding to the human target has most of the amino acid sequence replaced with a human sequence, and substantially retains the affinity and specificity of the parent non-human monoclonal antibody, and also it is less heterologous, therefore can be favorably applied to a human body. For example, the antibody or the antigen binding fragment according to the embodiments of the invention is capable of specifically binding to human B7-H3 after being humanized.

According to the embodiments of the invention, the non-human anti-B7-H3 antibody or fragment thereof is a murine anti-B7-H3 antibody or fragment thereof.

According to the embodiments of the invention, the step of utilizing CDR grafting technology to achieve the humanization of heavy chain variable region comprises:
(1) performing homology alignment using the heavy chain variable region sequence of a non-human anti-B7-H3 antibody or fragment thereof, and selecting a similar human antibody heavy chain template;
(2) grafting the VH CDRs 1, 2 and 3 of the non-human anti-B7-H3 antibody or fragment thereof into the human antibody heavy chain template;
(3) selecting a J region sequence according to the sequence of the VH CDR3, to achieve the humanization of heavy chain variable region and obtain the anti-human B7-H3 antibody or fragment thereof described above.

According to the embodiments of the invention, the step of utilizing CDR grafting technology to achieve the humanization of light chain variable region comprises:
(1) performing homology alignment using the light chain variable region sequence of a non-human anti-B7-H3 antibody or fragment thereof, and selecting a similar human antibody light chain template;
(2) grafting the VL CDRs 1, 2 and 3 of the non-human anti-B7-H3 antibody or fragment thereof into the human antibody light chain template;
(3) selecting a JK region sequence according to the sequence of the VL CDR3, to achieve the humanization of light chain variable region and obtain the anti-human B7-H3 antibody or fragment thereof described above.

According to still another aspect of the invention, the present disclosure provides a method for preparing an anti-human B7-H3 humanized antibody or fragment thereof. According to the embodiments of the invention, the method for preparing an anti-human B7-H3 humanized antibody or fragment thereof comprises: on the basis of the non-human anti-B7-H3 antibody or fragment thereof described above, utilizing CDR grafting technology to achieve the humanization of heavy chain variable region and/or the humanization of light chain variable region, in order to obtain the anti-human B7-H3 antibody or fragment thereof described above.

According to the embodiments of the invention, the step of utilizing CDR grafting technology to achieve the humanization of heavy chain variable region described above comprises:
(1) performing homology alignment using the heavy chain variable region sequence of the non-human anti-B7-H3 antibody or fragment thereof described above, and selecting a similar human antibody heavy chain template;
(2) grafting the VH CDRs 1, 2 and 3 of the non-human anti-B7-H3 antibody or fragment thereof into the human antibody heavy chain template;
(3) selecting a J region sequence according to the sequence of the VH CDR3, to achieve the humanization of heavy chain variable region and obtain the anti-human B7-H3 antibody or fragment thereof described above.

According to the embodiments of the invention, the step of utilizing CDR grafting technology to achieve the humanization of light chain variable region described above comprises:
(1) performing homology alignment using the light chain variable region sequence of the non-human anti-B7-H3 antibody or fragment thereof described above, and selecting a similar human antibody light chain template;
(2) grafting the VL CDRs 1, 2 and 3 of the non-human anti-B7-H3 antibody or fragment thereof into the human antibody light chain template;
(3) selecting a JK region sequence according to the sequence of the VL CDR3, to achieve the humanization of light chain variable region and obtain the anti-human B7-H3 antibody or fragment thereof described above.

According to the embodiments of the invention, the method for preparing an anti-human B7-H3 humanized antibody or fragment thereof further comprises: after the CDR grafting, performing affinity maturation, site-directed mutagenesis in the CDR sequences and/or chemical modification of the humanized antibody.

### Applications of the antibody

According to another aspect of the invention, the present disclosure provides a fusion molecule. According to the embodiments of the invention, the fusion molecule comprises:
(1) A first active element, which is a first targeting moiety comprising the anti-human B7-H3 antibody or fragment thereof described above;
(2) one or more second active elements, which comprise an effector element and/or a second targeting moiety;
wherein the first active element is linked to the second active element via a bond or a linker; and the one or more second active elements may be same or different.

According to another aspect of the invention, the present disclosure provides a polynucleotide. According to the embodiments of the invention, the polynucleotide encodes the anti-human B7-H3 antibody or fragment thereof described above or the fusion molecule described above. Thus, the polypeptide encoded by the polynucleotide is capable of specifically binding to B7-H3.

According to another aspect of the invention, the present disclosure provides a nucleic acid construct. According to the embodiments of the invention, the nucleic acid construct comprises the polynucleotide described above in the present disclosure.

According to another aspect of the invention, the present disclosure provides a host cell. According to the embodiments of the invention, the host cell comprises the polynucleotide or the nucleic acid construct described above in the present disclosure.

According to another aspect of the invention, the present disclosure provides a conjugate. According to the embodiments of the invention, the conjugate comprises a first active element, which is a first targeting moiety comprising the anti-human B7-H3 antibody or fragment thereof described above.

According to the embodiments of the invention, the conjugate further comprises at least one second active element, wherein the first active element is covalently linked to the second active element and the second active element comprises one or more effector moieties.

According to the embodiments of the invention, the effector moiety is a small molecule drug conjugate selected from the group consisting of one or more effector molecules or derivatives thereof in groups (1) to (3) as follows:
(1) small molecule compounds which are tubulin inhibitors: Maytansinoids, Monomethyl auristatin E, Monomethyl auristatin F, Monomethyl Dolastatin 10, Tubulysin and its derivatives, Cryptophycin and its derivatives, and Taltobulin;
(2) small molecule compounds which are topoisomerase inhibitors: PNU-159682 (the metabolite of Doxorubicin) and its derivatives, SN38 (the metabolite of Irinotecan) and its derivatives, and Exatecan;
(3) small molecule compounds which are DNA binding agents: PBD and its derivatives, and Duocarmycine and its derivatives.

According to another aspect of the invention, the present disclosure provides a composition. According to the embodiments of the invention, the composition comprises the anti-human B7-H3 antibody or fragment thereof described above, the fusion molecule described above, the polynucleotide described above, the nucleic acid construct described above, the host cell described above, or the conjugate described above; and optionally pharmaceutically acceptable excipient(s). Therefore, the composition is capable of specifically binding to B7-H3, and has a strong specificity and a good efficacy.

According to another aspect of the invention, the present disclosure provides a method for preparing an anti-human B7-H3 antibody or fragment thereof. According to the embodiments of the invention, the method for preparing an anti-human B7-H3 antibody or fragment thereof comprises:
(1) culturing the host cell described above under suitable conditions;
(2) separating and recovering an anti-human B7-H3 monoclonal antibody or fragment thereof, an anti-human B7-H3 chimeric antibody or fragment thereof, or an anti-human B7-H3 humanized antibody or fragment thereof.

Accordingly, the antibody prepared by the method according to the embodiments of the invention is capable of specifically binding to human B7-H3, and has a strong specificity.

According to another aspect of the invention, the present disclosure provides use of the anti-human B7-H3 antibody or fragment thereof described above, the fusion molecule described above, the polynucleotide described above, the nucleic acid construct described above, the host cell described above, the conjugate described above, and the composition described above in the preparation of an agent for diagnosing a disease associated with B7-H3 overexpression.

According to another aspect of the invention, the present disclosure provides use of the anti-human B7-H3 antibody or fragment thereof described above, the fusion molecule described above, the polynucleotide described above, the nucleic acid construct described above, the host cell described above, the conjugate described above, and the composition described above in the preparation of a medicament for treating a disease associated with B7-H3 overexpression.

According to the embodiments of the invention, the disease associated with B7-H3 overexpression is a solid tumor cancer.

According to the embodiments of the invention, the solid tumor cancer comprises: gastric cancer, colorectal cancer, liver cancer, pancreatic cancer, esophageal cancer, non-small cell carcinoma, prostate cancer, ovarian cancer, neuroblastoma, rhabdomyosarcoma, osteosarcoma, ewing sarcoma, wilms' tumor, and desmoplastic small round cell tumor.

The anti-human B7-H3 antibody or fragment thereof according to the embodiments of the invention is capable of specifically killing tumor cells, specifically binding to B7-H3 on the surface of recombinant cells and tumor cells, and contributes to a remarkable tumor killing effect in cells and animal models, and also no obvious adverse reactions are observed. Therefore, the defects of insufficient affinity and specificity of anti-human B7-H3 antibodies in the prior arts for the B7-H3 protein on the membrane surface which result in off-target effects and adverse reactions are overcome.

Further, according to yet another aspect of the invention, the present disclosure provides a method for treating a subject having a cancer. According to the embodiments of the invention, the method comprises administering to the subject a therapeutically effective amount of a composition, which is the composition described above.

According to the embodiments of the invention, the cancer is a solid tumor cancer.

According to the embodiments of the invention, the solid tumor cancer comprises: gastric cancer, colorectal cancer, liver cancer, pancreatic cancer, esophageal cancer, non-small cell carcinoma, prostate cancer, ovarian cancer, neuroblastoma, rhabdomyosarcoma, osteosarcoma, ewing sarcoma, wilms' tumor, and desmoplastic small round cell tumor.

To better understand the invention, certain terms are first defined. Other definitions are set forth throughout the detailed description.

The term "B7-H3" refers to B7 Homolog 3. B7-H3 is present in two forms: 2Ig-B7-H3 and 4Ig-B7-H3. In 2001, Chapoval et al. searched the databases of National Center for Biotechnology Information (NCBI) and Human Genome Sciences Inc. with extracellular domains of all published B7 family members. They identified a B7-like molecule in a human dendritic cells-derived cDNA library, and the nucleic acid sequence was confirmed using RT-PCR in human tumor cell line THP-1 and also DC libraries. The B7-H3 gene was found to encode a 316 aa protein containing a signal peptide, single variable- and constant-like immunoglobin domains, a transmembrane region, and a 45 aa cytoplasmic tail. By raising monoclonal antibodies to human dendritic cells, Steinberger et al. recognized a molecule that was induced on monocytes differentiated in vitro toward dendritic cells. Retroviral expression cloning identified the molecule as B7-H3 which was a type I membrane protein with four Ig-like domains, and the molecule identified was therefore named as 4Ig-B7-H3. RT-PCR analysis as well as Western blotting experiments pointed to 4Ig-B7-H3 as the prevalent form of B7-H3. As discussed by Steinberger et al., human B7-H3 is encoded on chromosome 15, and the 4Ig-B7-H3 is like to be the result of a duplication of the locus which encodes the B7-H3-IgV and -IgC exons.

The term "specific binding" refers to a non-random binding reaction between two molecules, such as the reaction between an antibody and an antigen to which the antibody is directed. The term "immunological binding" refers to a specific binding reaction occurring between an antibody molecule and an antigen for which the antibody is specific. The strength or affinity of an immunological binding interaction can be measured in terms of the equilibrium dissociation constant (KD) of the interaction, a smaller KD value indicating a higher affinity. The immunological binding properties between two molecules can be quantified using methods commonly known in the art. One method involves measuring the formation and dissociation rates of antigen binding site/antigen complex. Both the "association rate constant" (Ka or Kon) and the "dissociation rate constant" (Kd or Koff) for a particular antibody-antigen interaction can be calculated by concentration and the actual association and dissociation rates; see Malmqvist M, 1993, Nature, 361: 186-187. The ratio Kd/Ka is equal to the dissociation constant KD; see Davies DR et al., 1990, Annual Rev Biochem., 59: 439-473. KD, Ka and Kd values can be measured by any useful method. In preferred embodiments, the dissociation constant is measured using bioluminescence interferometry. In other preferred embodiments, the dissociation constant may be measured using surface plasmon resonance techniques (e.g., Biacore) or KinExa.

The term "antibody" as used herein is intended to encompass a full-length antibody and any antigen-binding fragment (i.e., an antigen-binding portion) or single chain thereof. The full-length antibody refers to a glycoprotein comprising at least two heavy (H) chains and two light (L) chains, the heavy and light chains being linked by disulfide bonds. Each heavy chain is composed of a heavy chain variable region (abbreviated as VH) and a heavy chain constant region. The heavy chain constant region is composed of three domains, i.e., CH1, CH2, and CH3. Each light chain is composed of a light chain variable region (abbreviated as VL) and a light chain constant region. The light chain constant region is composed of one domain CL. The VH and VL regions can further be divided into hypervariable regions termed Complementarity Determining Regions (CDRs) and more conserved Framework Regions (FRs) which separate the CDRs. Each VH and VL is composed of three CDRs and four FRs, arranged in the order FR1, CDR1, FR2, CDR2, FR3, CDR3, FR4 from the amino terminus to the carboxyl terminus. The variable regions of the heavy and light chains comprise binding domains that interact with an antigen. The constant regions of an antibody can mediate the binding of the immunoglobulin to a tissue or factor in a host, including various immune system cells (e.g., effector cells) and the first component of the classical complement system (C1q).

The term "monoclonal antibody" or "mAb" or "monoclonal antibody composition" refers to an antibody molecule preparation consisting of single molecules. A monoclonal antibody composition exhibits a single binding specificity and affinity for a particular epitope.

The term "antigen-binding fragment" of an antibody (or abbreviated as fragment of an antibody) as used herein refers to one or more fragments of an antibody that retain the ability to specifically bind to an antigen. It has been demonstrated that the antigen binding function of an antibody can be implemented by a fragment of the full-length antibody. Examples of a binding fragment encompassed within the "antigen binding portion" of an antibody include: (i) an Fab fragment, a monovalent fragment composed of VL, VH, CL and CH1; (ii) an F(ab')2 fragment, a bivalent fragment comprising two Fab fragments linked by a disulfide bridge at the hinge region; (iii) an Fd fragment composed of VH and CH1; (iv) an Fv fragment composed of the VL and VH of a single arm of an antibody; (v) a dAb fragment composed of VH (Ward et al, (1989) Nature 341: 544-546); (vi) an isolated Complementarity Determining Region (CDR); and (vii) a nanobody, a heavy chain variable region comprising a single variable domain and two constant domains. Furthermore, although the two domains VL and VH of an Fv fragment are encoded by different genes, they may be joined recombinantly via a synthetic linker to form a single protein chain, in which the VL and VH regions pair to form a monovalent molecule (referred to as a single chain Fc (scFv); see, e.g., Bird et al, (1988) Science 242: 423-426; and Huston et al., (1988) Proc. Natl. Acad. Sci. USA 85: 5879-5883). These single chain antibodies are also intended to be encompassed within the meaning of the term. These antibody fragments can be obtained using conventional techniques known to those skilled in the art, and can be functionally screened in the same manner as intact antibodies.

The antigen binding fragment of the invention includes those capable of specifically binding to an antigen molecule. Examples of an antibody binding fragment include, for example, but are not limited to, an Fab, an Fab', an F(ab')2, an Fv fragment, a single chain Fv (scFv) fragment, and a single domain fragment.

An Fab fragment contains the constant domain of the light chain and the first constant domain (CH1) of the heavy chain. An Fab' fragments differs from an Fab fragment by the addition of a few residues at the carboxyl terminus of the heavy chain CH1 domain including one or more cysteines from the antibody hinge region. An F(ab') fragment is produced by cleavage of the disulfide bond at the hinge cysteines of an F(ab')2 pepsin digestion product. Additional chemical couplings of antibody fragments are known to those of ordinary skill in the art. Fab and F(ab')2 fragments lack the fragment crystallizable (Fc) region of an intact antibody, clear more rapidly from the circulation of animals, and may have less non-specific tissue binding than an intact antibody (see, e.g. , Wahl et al, 1983, J. Nucl. Med. 24:316).

As is generally understood in the art, an "Fc" region is a fragment crystallizable constant region of an antibody that comprises no an antigen specific binding region. In IgG, IgA and IgD antibody isotypes, the Fc region consists of two identical protein fragments derived from the second and third constant domains of the two heavy chains of an antibody (CH2 and CH3 domains, respectively). The Fc regions of IgM and IgE contain three heavy chain constant domains (CH2, CH3, and CH4 domains) in each polypeptide chain.

The term "humanized antibody" mainly refers to an antibody expressed after engineering a murine monoclonal antibody using genetic cloning and DNA recombination technology. The antibody has most of the amino acid sequence replaced with a human sequence, and substantially retains the affinity and specificity of the parent murine monoclonal antibody, and also is less heterologous.

The term "chimeric antibody" refers to an antibody in which a portion of the heavy and/or light chain is identical with or homologous to corresponding sequences in an antibody derived from a particular species or belonging to a particular antibody class or subclass, while the remainder of the chain(s) is identical with or homologous to corresponding sequences in an antibody derived from another species or belonging to another antibody class or subclass, as well as fragments of such an antibody, so long as they exhibit the desired biological activity (see, e.g., U.S. Pat. No.4,816,567; and Morrison SL et al., Proc. Natl. Acad. Sci. USA, 81: 6851-6855, 1984). For example, the term "chimeric antibody" may include such an antibody (e.g., a human mouse chimeric antibody), in which the heavy and light chain variable regions of the antibody are derived from a first antibody (e.g., a murine antibody), while the heavy and light chain constant regions of the antibody are derived from a second antibody (e.g., a human antibody).

With respect to the term "heavy chain", a heavy chain (H chain) is about twice the size of the light chain, contains 450-550 amino acid residues, and has a molecular weight of about 55 or 75 kDa. Each H chain contains cyclic peptides formed via 4-5 intra-chain disulfide bonds. Different H chains have different antigenicity due to the different arrangement order of amino acid residues, number and position of disulfide bonds, and kind and number of the disulfide bonds, and can be classified into 5 types according to the difference of H chain antigenicity: µ chain, γ chain, α chain, δ chain, and ε chain. The entire immunoglobulin molecules made up of different H chains and L chains (κ or λ chains) are called IgM, IgG, IgA, IgD, and IgE respectively. The γ, α, and δ chains contain 4 peptides, and the µ and ε chains contain 5 cyclic peptides.

With respect to the term "light chain", a light chain (L chain) refers to a polypeptide chain which is smaller in molecular weight relative to the heavy chain in an immunoglobulin monomer molecule. The part within the 1/2 region near to the amino terminus (N-terminus) of each light chain having a much more diverse amino acid composition is the light chain variable region (VL), which is one part of the area where the Ig molecule binds to an antigen.

The remaining 1/2 region having a relatively stable amino acid composition and arrangement is the light chain constant region (CL). Light chain is present in two forms: κ and λ, due to some differences in the amino acid sequence within the light chain constant region.

The term "germ line", is also known as germline. Antibody-forming cells have all the genes encoding Ig molecules (i.e. a limited number of C genes and an unknown number of V genes), and the germ line is formed by a long time evolution and passed from parent to offspring by germ cells; and the heavy chain genes of human immunoglobulins are encoded by V-D-J-C gene segments, the light chain genes of human immunoglobulins are encoded by V-J-C gene segments, and the number of the gene segments varies. Human heavy chain genes are located on the long arm of chromosome 14, span about 1,100 kb, consist of four gene segments V, D, J and C, and comprise about 95 Va gene segments (belonging to 7 families, i.e., VHl to VH7), including 65 functional gene segments, 27 D gene segments, 6 JH gene segments and 9 CH gene segments. Va gene segments are located upstream, D gene segments are located between the VH and JH gene clusters, JH genes are located downstream of DH, separated about 7 Kb from the downstream C gene regions; CH genes are arranged in clusters, span about 200 kb; P and S genes are located immediately downstream of the JH gene segments, and downstream of C8 are Cγ, Cα, and Cε sequentially. Human light chain genes are grouped into λ and x genes, which are located on the long arm of chromosome 22 and the short arm of chromosome 2, respectively. There are about 40 functional VK gene segments, 5 functional J and 1 Cκ after Vc gene segments; about 30 Vλ gene segments, 4 Jλ gene segments and 4 Cλ gene segments.

The term "EC50" is also known as half maximal effect concentration, and refers to an antibody concentration causing 50% of the maximal effect.

The term "vector" or "nucleic acid construct" refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operably linked. Such vectors are referred to herein as "recombinant expression vectors" (or simply, "recombinant vectors"). In general, expression vectors of utility in recombinant DNA techniques are often in the form of plasmids. However, other forms of expression vectors can be encompassed, e.g., viral vectors (e.g., replication defective retroviruses, adenoviruses and adeno-associated viruses), which serve equivalent functions.

The term "nucleic acid molecule" is intended to include both DNA molecules and RNA molecules. The nucleic acid molecule may be single-stranded or double-stranded, and may be cDNA.

The term "polypeptide" refers to a chain comprising at least two consecutively linked amino acid residues, with no upper limit on the length of the chain. One or more amino acid residues in the protein may contain a modification such as, but not limited to, glycosylation, phosphorylation or a disulfide bond. A "protein" may comprise one or more polypeptides.

The term "host cell" refers to a cell in which a vector can be propagated and its DNA can be expressed, and the cell may be a prokaryotic cell or a eukaryotic cell. The term also encompasses any progeny of the subject host cell. It is understood that not all the progeny should be identical to a parent cell, since mutations may occur during replication and such progeny are encompassed.

The terms "cancer" and "tumor" refer to or describe the physiological condition of a mammal in which a population of cells is characterized by unregulated cell growth. Examples of cancer include, but are not limited to, carcinoma, lymphoma, blastoma, sarcoma, leukemia, and benign or malignant tumors. More specific examples of the cancer include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial carcinoma or uterine carcinoma, salivary gland carcinoma, kidney cancer, liver cancer, prostate cancer, vulval cancer, thyroid cancer, brain cancer, hepatic carcinoma, and various types of head and neck cancer, and neurofibromatosis type I or type II. Other examples of the cancer include those that are therapy resistant, refractory or metastatic.

The term "antibody-drug conjugate", abbreviated as "conjugate", consists of an antibody, a linker, and a drug, the linker being either a cleavable linker or a non-cleavable linker. Antibodies are globular proteins containing a series of amino acid sites that can be used to couple drug-linkers. Due to their tertiary and quaternary structures, only amino acids accessible to the solvent are available for coupling. In fact, high yields of coupling usually occur at the ε-amino group of a lysine residue or at the sulfhydryl group of a cysteine residue. The large number of lysine side chains on the surface of an antibody protein results in a large number of sites available for drug conjugation, resulting in the production of antibody-drug conjugates which are a mixture containing different numbers of drug conjugates (drug/antibody ratio, DAR) and different conjugation sites. The conjugation product provided by the present disclosure, although still as a mixture, has a narrow distribution range of DAR values compared to the antibody-drug conjugates obtained in traditional conjugation manner. The average DAR value is close to 4, closing to the optimum average range of DAR values (2-4) of antibody-drug conjugates. In addition, the conjugation product rarely contains naked antibody (DAR = 0), which component is not effective in killing cells. Also, the conjugation product contains no heavily coupled product (DAR = 8), which component will be cleared rapidly in vivo relative to the component having low DAR values. Therefore, the antibody-drug conjugate product provided by the present disclosure has a greatly improved heterogeneity.

The antibody to constitute the antibody-drug conjugate provided by the present disclosure would better retain its original antigen-binding ability in native state. Thus, the antibody in the present disclosure is capable of, preferably specifically, binding to its antigen. In order to develop an effective cellular level target for cancer diagnosis and treatment, researchers have sought to find transmembrane or other tumor-associated polypeptides. These targets are capable of being specifically expressed on the surface of one or more cancer cells, while not expressed or rarely expressed on the surface of one or more non-cancer cells. Typically, such tumor-associated polypeptides are more overexpressed on the surface of cancer cells relative to the surface of non-cancer cells. Identification of such tumor-related factors can greatly improve the specific targeting property of the antibody-based cancer treatment.

The antibodies provided by the present disclosure have a relatively higher binding capacity for B7-H3, and have an affinity constant KD value less than 5E-10 M. The anti-B7-H3 humanized antibodies hz9B11, hz10B4, and hz17A3 were capable of specifically binding to human B7-H3 protein, and their affinities (KD) were 2.19E-10 M, 1.42E-10 M, and 1.77E-10 M respectively, while the affinities (KD) of control antibodies MGC-018 and Enoblituzumab were 5.97E-10 M and 9.50E-10M respectively. The binding capacities provide a molecular basis for their good efficacies.

The antibodies provided by the present disclosure have good biological activities. Antibodies hz9B11, hz10B4, and hz17A3 were capable of effectively binding to the recombinant human B7-H3 on the cell surface, and their half maximal effective binding concentration (EC50) values were 0.08277 µg/ml, 0.1183 µg/ml, and 0.04407 µg/ml, respectively, superior to that of control antibody Enoblituzumab (EC50 value: 0.1906 µg/ml); and the EC50 values of hz9B11 and hz17A3 were close to that of control antibody MGC-018 (EC50 value: 0.05649 µg/ml). Further, hz9B11, hz10B4 and hz17A3 had good internalization capacities.

The antibodies provided by the present disclosure have good efficacies in vivo (mouse). In a subcutaneous transplantation tumor model of A431 cells in nude mice, the anti-B7-H3 antibody-drug conjugate (ADC) named hz10B4-M had an efficacy better than control antibody-drug conjugates MGC-018-M and Enoblituzumab-M, and hz17A3-M had an efficacy better than the control antibody-drug conjugate Enoblituzumab-M; while hz9B11-M showed a certain efficacy, weaker than those of hz10B4-M and hz17A3-M. In addition, no obvious toxic effect of the small molecule MMAE used for preparing the ADCs was observed; and the body weight of the animals in each experiment group steadily increased and there was no obvious difference in body weight between the experiment and control groups.

### BRIEF DESCRIPTION OF THE DRAWINGS

The above and/or additional aspects and advantages of the invention will become apparent and be readily appreciated from the description of the embodiments with reference to the following attached figures, in which:
FIG. 1: Binding of the hybridoma supernatants to the B7-H3 antigen detected by ELISA.
FIG. 2: Binding of the anti-human B7-H3 chimeric antibodies to different recombinant B7-H3 protein domains detected by ELISA.
FIG. 3: Binding of the anti-human B7-H3 chimeric antibodies to B7-H3 on the surface of 293/hB7-H3 cells detected by FACS.
FIG. 4: Binding of the anti-human B7-H3 chimeric antibodies to B7-H3 on the surface of A431 cells detected by FACS.
FIG. 5: ELISA curves showing the binding of the humanized antibodies to recombinant human B7-H3.
FIG. 6: ELISA curves showing the binding of the humanized antibodies to recombinant monkey B7-H3.
FIG. 7: ELISA curves showing the binding of the humanized antibodies to recombinant mouse B7-H3.
FIG. 8A: Analysis of the affinity of hz10B4 for recombinant human B7-H3 extracellular domain protein.
FIG. 8B: Analysis of the affinity of hz17A3 for recombinant human B7-H3 extracellular domain protein.
FIG. 8C: Analysis of the affinity of hz9B11 for recombinant human B7-H3 extracellular domain protein.
FIG. 8D: Analysis of the affinity of MGC-018 for recombinant human B7-H3 extracellular domain protein.
FIG. 8E: Analysis of the affinity of Enoblituzumab for recombinant human B7-H3 extracellular domain protein.
FIG. 9: Binding of the anti-human B7-H3 humanized antibodies to SKVO3 cells detected by FACS.
FIG. 10: Internalization rates of the anti-human B7-H3 humanized antibodies mediated by B7-H3 on cell surface.
FIG. 11: Detected killing activities of the anti-human B7-H3 antibody-drug conjugates on A431 cells.
FIG. 12: Detected killing activities of the anti-human B7-H3 antibody-drug conjugates on SKVO3 cells.
FIG. 13: Tumor volume change curves of the mice in the subcutaneous transplantation tumor model of A431 cells in nude mice.
FIG. 14: Weight change curves of the mice in the subcutaneous transplantation tumor model of A431 cells in nude mice.

### DETAILED DESCRIPTION OF PREFERRED EMBODIMENTS

The embodiments of the invention are described in detail below, and are illustrated in the accompanying drawings, wherein the same or similar reference numerals refer to the same or similar elements or elements having the same or similar functions throughout. The embodiments described below with reference to the accompanying drawings are exemplary and are only used to explain the invention, but should not be construed as a limitation on the invention.

It is to be noted that the terms "first" and "second" are only used for descriptive purposes, and should not be understood as indicating or implying a relative importance, or to implicitly indicating the number of technical features indicated. Thus, the features defined with "first" and "second" can explicitly or implicitly include one or more of the features. Further, in the description of the present disclosure, "multiple" means two or more, unless otherwise specifically indicated.

The solutions of the invention are illustrated below with reference to examples. It will be understood by those skilled in the art that the examples below are merely illustrative of the invention and not be regarded as limiting the scope of the invention. If no specific technology or conditions are indicated in the examples, the technology or conditions described in the literatures in the art (e.g., refer to Molecular Cloning: A Laboratory Manual (Third Edition), edited by J. Sambrook, and translated by Peitang HANG et al., Scientific Press) or product instructions shall be followed. The reagents or instruments used without the manufacturer's indication are conventional products that can be obtained commercially, for example, from Sigma Inc.

### Example 1: Preparation and screening of hybridoma cells producing anti-human B7-H3 antibodies

### 1.1 Hybridoma preparation

Immunization: Balb/c mice were immunized using a recombinant human B7-H3 extracellular domain protein (Accession No.: UniProtKB-Q5ZPR3, 1 aa-461 aa), and the serum titer was detected in 96-well ELISA plates coated with a recombinant human B7-H3-his protein (Accession No.: UniProtKB-Q5ZPR3, 1 aa-461 aa) through ELISA; the mice meeting the requirements of fusion were used for the next cell fusion.

Cell fusion and hybridoma preparation: mice with titer meeting the requirements were selected to receive final boost. 3 days later, the spleen of the mice were taken aseptically, suspensions of B lymphocytes were prepared and fused with SP2/0 myeloma cells. The fused cells were resuspended in HAT medium and then plated into 96-well cell culture plates which were then cultured in an incubator at 37 °C, 5% CO₂.

### 1.2 Binding screening of positive hybridomas

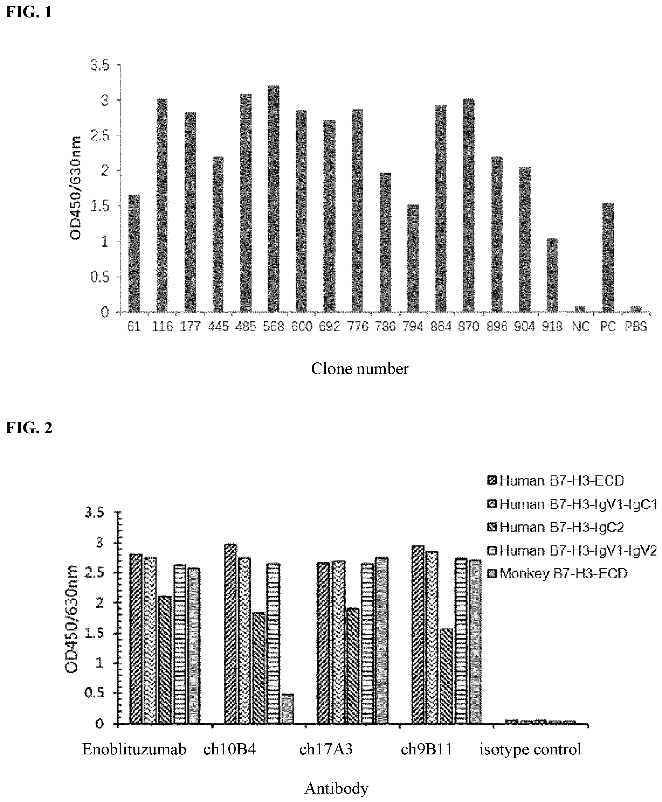

10-14 days after the fusion, ELISA plates were coated with a recombination human B7-H3-his extracellular domain protein (Accession No.: UniProtKB-Q5ZPR3, 1 aa-461 aa) (20 ng/ml) at 4 °C overnight. Washed with PBS three times, the plates were blocked with 4% skimmed milk powder-PBS at room temperature for 1 hr. Subsequently, the plates were washed with PBS three times, and culture supernatants of the hybridoma clones were added into the plates and incubated at room temperature for 1 hr. Following controls were set up: (1) Positive Control (PC): post-immunization mouse serum (1: 1000 diluted with PBS); and (2) Negative Control (NC): pre-immunization mouse serum (1: 1000 diluted with PBS). The plates were washed with PBST (0.05% Tween 20-PBS) three times and washed with PBS twice; and then HRP conjugated goat anti-mouse IgG (Fcy) was added into the plates and incubated at 37 °C for 0.5 hr. Again, the plates were washed with PBST (0.05% Tween 20-PBS) three times and TMB substrate was added for color development in dark for 15-30 min. ELISA stopping solution was added to stop the reaction; and absorbance at 450 nm was read on a microplate reader. The top 16 clones with readings ordered from highest to lowest were selected and their culture supernatants were subjected to the second ELISA for confirmation. Finally, Clones Nos. 445 (9B11), 485 (10B4), and 870 (17A3) were selected as candidate clones for sequence cloning. The ELISA results are shown in FIG. 1.

### Example 2: Sequencing of murine anti-human B7-H3 antibodies

The monoclonal hybridoma cells 9B11, 10B4, and 17A3 secreting anti-human B7-H3 antibodies were subject to expansion culture, and total RNA of the cells was extracted using TRIzol kit (Cat: 15596026, Invitrogen) according to the steps described in the instructions provided in the kit; the total RNA of the hybridoma cells obtained was reverse transcribed to cDNA using M-MuLV reverse transcriptase (Cat: M0253S, NEB); and the sequences of antibody light chain variable region IgVL (κ) and heavy chain variable region VH were amplified using degenerate primers and Phusion kit (Cat: E0553L, NEB). PCR amplification products were purified using a gel recovery kit (Cat: AP-GX-250, Axygen); and linked to T-vector using a T vector cloning kit (Cat: ZC205, ZOMANBIO) according to the instructions provided in the kit, and transformed into competent E. coli cells. After strain amplification and plasmid extraction, variable region sequences of the monoclonal antibodies were obtained by DNA sequencing. The heavy chain variable region sequence of the murine antibody 9B11 is as shown in SEQ ID NO: 1 and the light chain variable region sequence of the murine antibody 9B11 is as shown in SEQ ID NO: 2. The heavy chain variable region sequence of the murine antibody 10B4 is as shown in SEQ ID NO: 3 and the light chain variable region sequence of the murine antibody 10B4 is as shown in SEQ ID NO: 4. The heavy chain variable region sequence of the murine antibody 17A3 is as shown in SEQ ID NO: 5 and the light chain variable region sequence of the murine antibody 17A3 is as shown in SEQ ID NO: 6.

### Example 3: Preparation of anti-human B7-H3 chimeric antibodies and control antibodies

The light and heavy chain sequences of control antibodies Enoblituzumab and MGC-018 were fully synthesized, and cloned into a eukaryotic transient-expression vector respectively to obtain plasmids expressing the light and heavy chains of the control antibodies. The plasmids were transformed into E. coli cells for expansion, and a large number of plasmids containing the light and heavy chains of the control antibodies respectively were obtained through recovery. The plasmids containing the light chain and heavy chain of the control antibodies were in turn transfected into HEK293 cells respectively using 293fectin (Cat.: 12347019, Gibco) transfection reagent following the manufacturer's instructions for recombinant expression. 5-6 days after the cell transfection, culture supernatants were taken and purified through ProA affinity chromatography column to obtain the control antibodies. The amino acid sequences of the control antibody Enoblituzumab were derived from WHO Drug Information (Vol. 30, No. 4, 2016), and the amino acid sequence of the heavy chain is as shown in SEQ ID NO: 7, and the amino acid sequence of the light chain is as shown in SEQ ID NO: 8. The amino acid sequences of the control antibody MGC-018 were derived from patent publication WO2017180813, and the amino acid sequence of the heavy chain is as shown in SEQ ID NO: 9, and the amino acid sequence of the light chain is as shown in SEQ ID NO: 10.

The light chain variable region and heavy chain variable region genes of the murine antibodies 9B11, 10B4 and 17A3 obtained by cloning as above, with cleavage sites of restriction enzymes introduced by PCR, were respectively cloned into the upstream of gene encoding human-kappa light chain constant region and the upstream of gene encoding human IgG1 heavy chain constant region carried in eukaryotic transient-expression vectors. Plasmids expressing human-murine chimeric light chain (pKN019-ch9B11L, pKN019-ch10B4L, and pKN019-ch17A3L) and plasmids expressing human-murine chimeric heavy chain (pKN025-ch9B11H, pKN025-ch10B4H, and pKN025-ch17A3H) were obtained and transformed into E. coli cells for expansion, and a large number of plasmids containing human-murine chimeric light chain and heavy chain respectively were obtained through recovery. The plasmids containing the light chains and heavy chains of chimeric antibodies 9B11, 10B4 and 17A3 were in turn transfected into HEK293 cells respectively using 293fectin (Cat.: 12347019, Gibco) transfection reagent following the manufacturer's instructions for recombinant expression. 5-6 days after cell transfection, culture supernatants were taken and purified through ProA affinity chromatography columns to obtain chimeric antibodies 9B11, 10B4 and 17A3 (ch9B11, ch10B4, and ch17A3).

### Example 4: Analysis of affinities of chimeric antibodies

Affinities of the antibodies were determined by an assay including capturing Fc fragments of the antibodies with anti-human IgG Fc capture (AHC) biosensors using Octet QKe system instrument from Fortebio. For the assay, each of the chimeric antibodies ch9B11, ch10B4, ch17A3 and the control antibody Enoblituzumab was diluted to 4 µg/ml in PBS, and was allowed to flow through the surface of an ARC biosensor (Cat.: 18-0015, PALL) for 120 s. Recombinant human B7-H3 extracellular domain protein (Accession No.: UniProtKB-Q5ZPR3, 1 aa-461 aa, C-his tag) (60 nM) was used as a mobile phase. The association time was 300 s and the dissociation time was 300 s. When the assay was finished, data from which the response values of blank control had been deducted were fitted to a 1:1 Langmuir binding model using software, and then kinetic constants for antigen-antibody binding were calculated. Kinetic constants are shown in Table 1 below, and the results in Table 1 show that all the clones were cloned correctly and capable of binding to the recombinant human B7-H3 protein.

**TABLE 1. Detection results of the affinities of the chimeric antibodies for recombinant human B7-H3 protein**

| Clone | Human B7-H3 ECD | | |
|---|---|---|---|
| | KD (M) | Kon(1/Ms) | Kdis(1/s) |
| ch9B11 | 1.20E-10 | 1.17E+06 | 1.41E-04 |
| ch10B4 | 4.35E-10 | 6.02E+05 | 2.62E-04 |
| ch17A3 | 2.69E-10 | 9.65E+05 | 2.59E-04 |
| Enoblituzumab | 7.73E-10 | 3.31E+05 | 2.56E-04 |

### Example 5: Binding of chimeric antibodies to different recombinant B7-H3 proteins and different domains detected by ELISA

Plates were coated with recombinant human B7-H3-his protein (Accession No.: UniProtKB-Q5ZPR3, 1 aa-461 aa), recombinant human B7-H3 IgV1-IgV2-his extracellular domain protein (Accession No.: UniProtKB-Q5ZPR3, 1 aa-362 aa), recombinant human B7-H3 IgV1-IgC1-his extracellular domain protein (Accession No.: UniProtKB-Q5ZPR3, 1 aa-238 aa), recombinant human B7-H3 IgC2-mFc extracellular domain protein (Accession No.: UniProtKB-Q5ZPR3, 363 aa-456 aa), and recombinant monkey B7-H3-his extracellular domain protein (Accession No.: XP-005560056.1, 1 aa-465 aa) respectively overnight at 4 °C, each protein having a concentration of 0.2 µg/ml in 100 µl. Then the plates were blocked with 5% BSA in a thermostatic incubator at 37 °C for 60 min, and afterwards were washed 3 times with PBST. 100 µl of each of ch9B11, ch10B4, and ch17A3, as well as control antibody Enoblituzumab and an isotype control antibody (NC) having a concentration of 0.5 µg/ml were added and then reacted in a thermostatic incubator at 37 °C for 60 min. After the incubation, the plates were washed with PBST 4 times, and 1:5000 diluted HRP-anti-human Fc (Cat.: 109-035-098, Jackson Immuno Research) was added into the plates for reaction for 45 min. TMB (Cat.: ME142, GalaxyBio, Beijing) substrate was added for color development for 15 min, and then 2 M HCl was added to stop the reaction. Absorbances at 450 nm and at 630 nm (as reference wavelength) were read, and A450nm-630nm values of the wells in the plates were recorded.

As detected by ELISA, ch9B11, ch10B4, and ch17A3 as well as control antibody Enoblituzumab all bound to the recombination human B7-H3 domain proteins and the recombinant monkey B7-H3 extracellular domain protein (FIG. 2); among the antibodies, the binding activity to the monkey B7-H3 of ch10B4 was obviously weaker than those of other antibodies, indicating a weak cross-binding activity; and all the binding of the antibodies to B7H3-IgC2 was slightly weaker than the binding of them to other domains, and ch9B11 was the most obvious one.

### Example 6: Binding of anti-human B7-H3 chimeric antibodies to recombinant human B7-H3 protein on the surface of 293 cells detected by FACS

The construction of 293 cell line transiently expressing human B7-H3: HEK293 cells were inoculated into a shake flask at a density of 6 × 10⁵ cells/ml in a 10 ml volume and then subjected to a shaking culture at 130 rpm in a shaking incubator at 37 °C, 5% CO₂ for 24 h. An expression plasmid encoding full-length human B7-H3 (Accession No.: UniProtKB-Q5ZPR3, 1 aa-534 aa) and 293fectin (Cat: 12347019, Gibco) in a ratio of 1: 1.2 were allowed to stand in 0.5 mL Opti-MEM (Cat: 11058021, Gibco) medium respectively for 5 min, and then were mixed and let stand at room temperature for 15 min. Then the mixture was added to the HEK293 cells, which were then subjected to a shaking culture at 130 rpm in a shaking incubator at 37 °C, 5% CO₂ for 48 h. The obtained cells i.e. 293/hB7-H3 cells were used for the detection via FACS as follows.

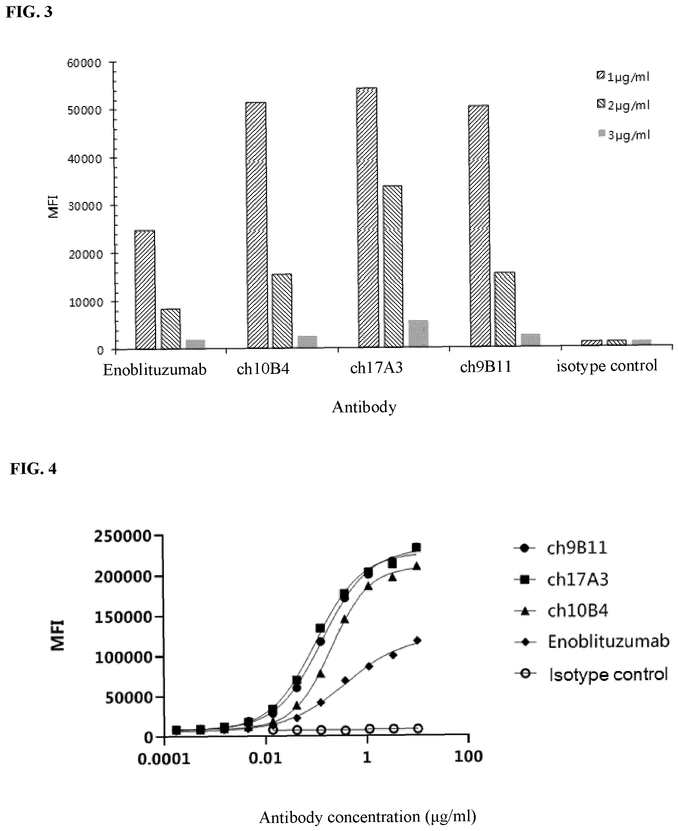

Suspensions of 293 cells recombinantly expressing human B7-H3 (293/hB7-H3 cells) were incubated with the chimeric antibodies (ch9B11, ch10B4, and ch17A3; at concentrations of 1 µg/ml, 0.1 µg/ml and 0.01µg/ml respectively) at 37 °C for 30 min. Controls as follows were set: (1) Positive Control (PC): control antibody Enoblituzumab; (2) Negative Control (NC): an isotype control antibody. The cells were washed 3 times with PBS, and 1:200 diluted goat anti-human IgG-FITC (Cat.: F9512, Sigma) was added into the cells which then were incubated for 30 min. Then the cells were washed 3 times with PBS again, and the Mean Fluorescence Intensity (MFI) of the cells was measured by a flow cytometer (model B49007AD, SNAW31211, BECKMAN COULTER) to detect the binding abilities of the chimeric antibodies to the human B7-H3 protein on the surface of the 293 cells. As detected by FACS, ch9B11, ch10B4, and ch17A3 all could bind to the recombination human B7-H3 protein on the surface of the 293 cells, with binding capacities superior to that of the control antibody Enoblituzumab (FIG. 3).

### Example 7: Binding of anti-human B7-H3 chimeric antibodies to A431 cells (human skin squamous carcinoma cells) detected by FACS

Suspensions of A431 cells naturally expressing human B7-H3 were incubated with the chimeric antibodies (ch9B11, ch10B4, and ch17A3; diluted in a 3-fold gradient from a concentration of 10 µg/ml, 11 concentrations in total) at 37 °C for 30 min. Controls as follows were set: (1) Positive Control (PC): control antibody Enoblituzumab; (2) Negative Control (NC): an isotype control antibody. The cells were washed 3 times with PBS, and 1:200 diluted goat anti-human IgG-FITC (Cat.: F9512, Sigma) was added into the cells which then were incubated for 30 min. Then the cells were washed 3 times with PBS again, and the Mean Fluorescence Intensity (MFI) of the cells was measured by a flow cytometer (model B49007AD, SNAW31211, BECKMAN COULTER) to detect the binding abilities of the chimeric antibodies to the B7-H3 protein on the surface of A431 cells. As detected by FACS, ch9B11, ch10B4, and ch17A3 all could bind to the B7-H3 protein on the surface of the A431 cells, with binding capacities superior to that of the control antibody Enoblituzumab. Respective half maximal effective binding concentration (EC50) values of the antibodies are shown in Table 2, and the binding is shown in FIG. 4.

**TABLE 2. EC50 values of the binding of the anti-human B7-H3 chimeric antibodies to A431 cells detected by FACS**

| | ch9B11 | ch17A3 | ch10B4 | Enoblituzumab |
|---|---|---|---|---|
| EC50 (µg/ml) | 0.1366 | 0.1008 | 0.2028 | 0.3744 |

### Example 8: Humanization and mutation design of anti-human B7-H3 monoclonal antibodies

### 8.1 Humanization of murine monoclonal antibody 9B11

### (1) CDR grafting

Firstly, the heavy chain sequence of the murine antibody was comprehensively analyzed, and complementarity-determining regions (CDRs) accounting for antigen-antibody binding and framework regions supporting the conserved three-dimensional conformation of the antibody were determined. Subsequently according to homology alignment results, the most similar human template VH1(1-03) was selected as the basic template, and CDR grafting was performed in combination with the full-sequence BLAST results; and for the CDR grafting, JH6 (wgqgtTvtvss) was selected as the J region sequence based on the sequence of CDR3 (STTTATFYWYFDV; SEQ ID NO: 42), and then the humanization in the framework regions of the heavy chain variable region (VH) of 9B11 was achieved. In a similar way, the most similar human templates VK III (A27) and VKVI (A26) were selected as the basic templates, and CDR grafting was performed in combination with the full-sequence BLAST results; and for the CDR grafting, JK2 (FGQGTKLEIK) was selected as the JK region sequence based on the sequence of CDR3 (QQSHSWPYT; SEQ ID NO: 45), and then the humanization in the framework regions of the light chain was achieved. The amino acid sequence of the humanized heavy chain variable region hz9B11_VH1 with CDRs grafted from antibody 9B11 is as shown in SEQ ID NO: 11; and the amino acid sequence of the humanized light chain variable region hz9B11_VL1 with CDRs grafted from antibody 9B11 is as shown in SEQ ID NO: 12, and the amino acid sequence of the humanized light chain variable region hz9B11_VL2 with CDRs grafted from antibody 9B11 is as shown in SEQ ID NO: 13.

### (2) Mutation design in CDRs

According to sequence characteristics of the murine antibody 9B11, mutations in the sequences of the humanized heavy and light chain variable regions with CDRs grafted were designed and the mutation sites are shown in Table 3 below.

**TABLE 3. Design of humanized sequences of 9B11**

| SEQ ID NO: | hz9B11_VL | | SEQ ID NO: | hz9B11_VH | |
|---|---|---|---|---|---|
| 12 | hz9B11_VL1 | CDRs Grafted | 11 | hz9B11_VH1 | CDRs Grafted |
| 14 | hz9B11_VL3 | Y49K | 17 | hz9B11_VH2 | Q1E |
| 15 | hz9B11_VL4 | Y87F | | | |
| 16 | hz9B11_VL5 | Y49K, Y87F | | | |

| | | | | | |
|---|---|---|---|---|---|
| Note: the number indicates the position of the mutated amino acid, the letter before the number indicates the amino acid residue before the mutation, and the letter after the number indicates the amino acid residue after the mutation, e.g. Y49K indicates the amino acid at position 49 is mutated from Y to K. | | | | | |

### 8.2 Humanization of murine monoclonal antibody10B4

### (1) CDR grafting

Firstly, the heavy chain sequence of the murine antibody was comprehensively analyzed, and complementarity-determining regions (CDRs) accounting for antigen-antibody binding and framework regions supporting the conserved three-dimensional conformation of the antibody were determined. Subsequently according to homology alignment results, VH1(1-03) was selected as the basic template, and CDR grafting was performed in combination with the full-sequence BLAST results; and for the CDR grafting, JH6 (WGQGTTVTVSS) was selected as the J region sequence based on the sequence of CDR3 (KGKDYFDWYFDV; SEQ ID NO: 48), and then the humanization in the framework regions of the heavy chain variable region (VH) of 10B4 was achieved. According to homology alignment results, the most similar human template VK I (012) was selected as the basic template, and CDR grafting was performed in combination with the full-sequence BLAST results; and for the CDR grafting, JK4 (FGGGTKVEIK) was selected as the JK region sequence based on the sequence of CDR3 (QQWSSNPLT; SEQ ID NO: 51), and then the humanization in the framework regions of the light chain was achieved. The amino acid sequence of the humanized heavy chain variable region hz10B4_VH1 with CDRs grafted from antibody 10B4 is as shown in SEQ ID NO: 18; and the amino acid sequence of the light chain variable region hz10B4_VL1 with CDRs grafted from antibody 10B4 is as shown in SEQ ID NO: 19.

### (2) Mutation design in CDRs

According to sequence characteristics of the murine antibody 10B4, mutations in the sequences of the humanized heavy and light chain variable regions with CDRs grafted were designed and the mutation sites are shown in Table 4 below.

**TABLE 4 Design of humanized sequences of 10B4**

| SEQ ID NO: | hz10B4_VL | | SEQ ID NO: | hz10B4_VH | |
|---|---|---|---|---|---|
| 19 | hz10B4_VL1 | CDRs Grafted | 18 | hz10B4_VH1 | CDRs Grafted |
| 20 | hz10B4_VL2 | M4L | 21 | hz10B4_VH2 | Q1E, D89E |
| 58 | hz10B4_VL3 | N93A | 22 | hz10B4_VH3 | Q1E, N54A, N55S, D89E |
| 59 | hz10B4_VL4 | S92A, N93S | 23 | hz10B4_VH4 | Q1E, N55Q, D89E |
| 60 | hz10B4_VL5 | N93 Q | | | |

| | | | | | |
|---|---|---|---|---|---|
| Note: the number indicates the position of the mutated amino acid, the letter before the number indicates the amino acid residue before the mutation, and the letter after the number indicates the amino acid residue after the mutation, e.g. M4L indicates the amino acid at position 4 in the light chain variable region of hz10B4_VL1 is mutated from M to L. | | | | | |

### 8.3 Humanization of murine monoclonal antibody17A3

### (1) CDR grafting

Firstly, the heavy chain sequence of the murine antibody was comprehensively analyzed, and complementarity-determining regions (CDRs) accounting for antigen-antibody binding and framework regions supporting the conserved three-dimensional conformation of the antibody were determined. Subsequently according to homology alignment results, the most similar human template VH1(1-02) was selected as the basic template, and CDR grafting was performed in combination with the full-sequence BLAST results; and for the CDR grafting, JH4 (WGQGTLVTVSS) was selected as the J region sequence based on the sequence of CDR3 (GITAWGTRYYAMDY), and then the humanization in the framework regions of the heavy chain variable region (VH) of 17A3 was achieved. According to homology alignment results, VK III (A27) and VKVI (A26) were selected as the basic templates, and CDR grafting was performed in combination with the full-sequence BLAST results; and for the CDR grafting, JK4 (FGGGTKVEIK) was selected as the JK region sequence based on the sequence of CDR3 (QQSYSWPLT), and then the humanization in the framework regions of the light chain was achieved. The amino acid sequence of the humanized heavy chain variable region hz17A3_VH1 with CDRs grafted from antibody 17A3 is as shown in SEQ ID NO: 24; and the amino acid sequence of the light chain variable region hz17A3_VL1 with CDRs grafted from antibody 17A3 is as shown in SEQ ID NO: 25, and the amino acid sequence of the light chain variable region hz17A3_VL2 with CDRs grafted from antibody 17A3 is as shown in SEQ ID NO: 26.

### (2) Mutation design in CDRs

According to sequence characteristics of the murine antibody 17A3, mutations in the sequences of the humanized heavy and light chain variable regions with CDRs grafted were designed and the mutation sites are shown in Table 5 below.

**TABLE 5. Design of humanized sequences of 17A3**

| SEQ ID NO: | hz17A3_VL | | SEQ ID NO: | hz17A3_VL | | SEQ ID NO: | hz17A3_VH | |
|---|---|---|---|---|---|---|---|---|
| 25 | hz17A3_VL1 | CDRs Grafted | 26 | hz17A3_VL2 | CDRs Grafted | 24 | hz17A3_VH1 | CDRs Grafted |
| 27 | hz17A3_VL3 | Y49K | 32 | hz17A3_VL8 | Y49K | 34 | hz17A3_VH2 | M48I |
| 28 | hz17A3_VL4 | Y87F | 33 | hz17A3_VL9 | Y87F | 35 | hz17A3_VH3 | Q1E, D89E |
| 29 | hz17A3_VL5 | N31S, Y49K | | | | 36 | hz17A3_VH4 | Q1E, N52S, D89E |
| 30 | hz17A3_VL6 | Y49K, N54S | | | | 37 | hz17A3_VH5 | Q1E, N57S, D89E |
| 31 | hz17A3_VL7 | N31S, Y49K, N54S | | | | | | |

| | | | | | | | | |
|---|---|---|---|---|---|---|---|---|
| Note: the number indicates the position of the mutated amino acid, the letter before the number indicates the amino acid residue before the mutation, and the letter after the number indicates the amino acid residue after the mutation, e.g. Y49K indicates the amino acid at position 49 in the light chain variable region of hz17A3_VL1 is mutated from Y to K. | | | | | | | | |

### Example 9: Recombinant expression of anti-human B7-H3 humanized monoclonal antibodies

The sequences of the light chain variable regions and heavy chain variable regions (hz9B11_VL1, hz9B11_VL2, hz9B11_VH1, hz10B4_VL1, hz10B4_VH1, hz17A3_VL1, hz17A3_VL2, and hz17A3_VH1) obtained by the humanization designs based on the antibodies 9B11, 10B4, and 17A3 were fully synthesized. The humanized heavy chain variable regions were cloned by enzyme digestion into eukaryotic transient-expression vector pKN041 at the upstream of the gene encoding the heavy chain constant region of human IgG1, and the amino acid sequence of the heavy chain constant region is as shown in SEQ ID NO: 38; and the humanized light chain variable regions were cloned by enzyme digestion into eukaryotic transient-expression vector pKN019 at the upstream of the gene encoding human Cκ light chain, and the amino acid sequence of the light chain constant region is as shown in SEQ ID NO: 39; thereby plasmids expressing the humanized light chains and heavy chains based on the antibodies 9B11, 10B4, and 17A3 were constructed. According to the mutation designs, site-directed mutagenesis was performed in the plasmids expressing the humanized light chains and heavy chains respectively using StarMut gene Site-directed Mutagenesis Kit (Cat.: T111-01, GenStar). The mutated plasmids were transformed into E.coli cells for expansion, and plasmids expressing the humanized and mutated light and heavy chains based on the antibodies 9B11, 10B4, and 17A3 were obtained. All the plasmids containing various humanized light and heavy chains based on the antibodies 9B11, 10B4, and 17A3 were combined, as shown in Tables 6-8, and transfected into HEK293 cells using 293fectin (Cat.: 12347019, Gibco) transfection reagent following the manufacturer's instructions for recombinant expression. 5-6 days after cell transfection, culture supernatants were purified through ProA affinity chromatography column to obtain different humanized antibodies. Affinities of the antibodies were determined by an assay including capturing Fc fragments of the antibodies with anti-human IgG Fc capture (AHC) biosensors using Octet QKe system instrument from Fortebio. For the assay, each of the humanized antibodies and the chimeric antibodies based on the antibodies 9B11, 10B4, and 17A3 was diluted to 4 µg/ml in PBS, and was allowed to flow through the surface of an ARC biosensor (Cat.: 18-0015, PALL) for 300 s. Recombinant human B7-H3-his protein (Accession No.: UniProtKB-Q5ZPR3, 1 aa-461 aa) was used as a mobile phase, and its concentration was 60 nM. The association time was 100 s and the dissociation time was 300 s. When the assay was finished, data from which the response values of blank control had been deducted were fitted to a 1:1 Langmuir binding model using software, and then kinetic constants for antigen-antibody binding were calculated. The light and heavy chain combinations and the affinity constant KD values of the humanized and the mutant antibodies based on the antibodies 9B 11, 10B4, and 17A3 are shown in Tables 6-8 respectively.

The detection results of the affinities of those mutant antibodies showed that the antibody affinity would change greatly with the mutations at different sites. Among the antibodies, the antibody hz9B11-6 exhibiting an affinity (KD) of 1.55E-10 M was selected and named as "hz9B11"; the antibody hz10B4-7 exhibiting an affinity (KD) of 1.61E-10 M was selected and named as "hz10B4"; and the antibody hz17A3-14 exhibiting an affinity (KD) of 3.01E-10 M was selected and named as "hz17A3", and the three antibodies were selected for functional verification in subsequent studies.

**TABLE 6. Combinations of the humanized light and heavy chains based on 9B11 and affinity constant (KD) values**

| Name | Amino acid sequences of the antibody (SEQ ID NOs:) | | KD value (M) |
|---|---|---|---|
| | Light chain variable region | Heavy chain variable region | |
| ch9B 11 | 2 | 1 | 9.98E-11 |
| hz9B 11-1 | 12 | 11 | 4.52E-9 |
| hz9B 11-2 | 12 | 17 | 6.28E-9 |
| hz9B 11-3 | 13 | 11 | 1.18E-10 |
| hz9B 11-4 | 13 | 17 | 1.41E-10 |
| hz9B 11-5 | 14 | 11 | 1.21E-10 |
| hz9B 11-6 | 14 | 17 | 1.55E-10 |
| hz9B 11-7 | 15 | 11 | 3.07 E-09 |
| hz9B 11-8 | 15 | 17 | 4.51 E-09 |
| hz9B 11-9 | 16 | 11 | 1.07 E-10 |
| hz9B 11-10 | 16 | 17 | 1.39 E-10 |

**TABLE 7. Combinations of the humanized light and heavy chains based on 10B4 and affinity constant (KD) values**

| Name | Amino acid sequences of the antibody (SEQ ID NOs:) | | KD value (M) |
|---|---|---|---|
| | Light chain variable region | Heavy chain variable region | |
| ch10B4 | 4 | 3 | 3.55E-10 |
| hz10B4-1 | 19 | 18 | 8.27E-10 |
| hz10B4-2 | 19 | 21 | 1.77E-10 |
| hz10B4-3 | 19 | 22 | 3.41E-10 |
| hz10B4-4 | 19 | 23 | 5.01E-10 |
| hz10B4-5 | 20 | 18 | 9.98E-10 |
| hz10B4-6 | 20 | 21 | 1.31E-10 |
| hz10B4-7 | 20 | 22 | 1.61E-10 |
| hz10B4-8 | 20 | 23 | 2.11E-10 |
| hz10B4-9 | 58 | 22 | 3.52E-10 |
| hz10B4-10 | 59 | 22 | 3.81E-10 |
| hz10B4-11 | 60 | 22 | 4.22E-10 |
| hz10B4-12 | 58 | 23 | 3.32E-10 |
| hz10B4-13 | 59 | 23 | 3.51E-10 |
| hz10B4-14 | 60 | 23 | 5.23E-10 |

**TABLE 8. Combinations of the humanized light and heavy chains based on 17A3 and affinity constant (KD) values**

| Name | Amino acid sequences of the antibody (SEQ ID NOs:) | | KD value (M) |
|---|---|---|---|
| | Light chain variable region | Heavy chain variable region | |
| ch17A3 | 6 | 5 | 3.67E-10 |
| hz17A3-1 | 25 | 24 | 5.41E-10 |
| hz17A3-2 | 25 | 34 | 7.71E-10 |
| hz17A3-3 | 26 | 24 | 5.98E-10 |
| hz17A3-4 | 26 | 34 | 8.99E-10 |
| hz17A3-5 | 27 | 35 | 2.58E-10 |
| hz17A3-6 | 27 | 36 | 4.01E-10 |
| hz17A3-7 | 27 | 37 | 4.22E-10 |
| hz17A3-8 | 28 | 35 | 4.62E-10 |
| hz17A3-9 | 28 | 36 | 4.31E-10 |
| hz17A3-10 | 28 | 37 | 4.01E-10 |
| hz17A3-11 | 29 | 35 | 2.99E-10 |
| hz17 A3-12 | 29 | 36 | 5.73E-10 |
| hz17A3-13 | 29 | 37 | 6.99E-10 |
| hz17A3-14 | 30 | 35 | 3.01E-10 |
| hz17A3-15 | 30 | 36 | 3.71E-10 |
| hz17A3-16 | 30 | 37 | 2.99E-10 |
| hz17A3-17 | 31 | 35 | 3.28E-11 |
| hz17A3-18 | 31 | 36 | 1.72E-10 |
| hz17A3-19 | 31 | 37 | 4.11E-10 |
| hz17A3-20 | 32 | 35 | 2.89E-10 |
| hz17A3-21 | 32 | 36 | 4.33E-10 |
| hz17A3-22 | 32 | 37 | 4.79E-10 |
| hz17A3-23 | 33 | 35 | 2.58E-09 |
| hz17A3-24 | 33 | 36 | 1.91E-09 |
| hz17A3-25 | 33 | 37 | 4.88E-09 |

### Example 10: Species specificity study on the binding of anti-B7-H3 humanized antibodies to B7-H3 detected by ELISA

Plates were coated with recombinant human B7-H3-his extracellular domain protein (Accession No.: UniProtKB-Q5ZPR3, 1 aa-461 aa), recombinant cynomolgus B7-H3-his protein (Cat.: 90806-C08H, Beijing Yiqiao Shenzhou Science and Technology Co., Ltd.) and recombinant mouse B7-H3-his protein (Cat.: CM83, Beijing Yiqiao Shenzhou Science and Technology Co., Ltd.) respectively overnight at 4 °C, each protein having a coating concentration of 1 µg/ml. Washed with PBS 3 times, the plates were blocked with the added 5% BSA PBS at 37 °C for 60 min, and then washed 3 times with PBST. Different dilutions of hz10B4 (diluted in a 3-fold gradient from a starting concentration of 10 µg/ml, 12 concentrations in total), hz17A3 (diluted in a 3-fold gradient from a starting concentration of 3 µg/ml, 12 concentrations in total), hz9B11 (diluted in a 3-fold gradient from a starting concentration of 10 µg/ml, 12 concentrations in total), Enoblituzumab (diluted in a 3-fold gradient from a starting concentration of 10 µg/ml, 12 concentrations in total), and MGC-018 (diluted in a 3-fold gradient from a starting concentration of 10 µg/ml, 12 concentrations in total) were added into the plates. The plates were incubated at 37 °C for 60 min, and then were washed 4 times with PBST. 1:5000 diluted HRP-anti-human Fc (Cat.: 109-035-098, Jackson Immuno Research) was added into the plates for incubation at 37 °C for 30 min. Afterwards, the plates were washed with PBST 4 times, and TMB substrate was added for color development. After incubation at 37 °C for 10 min, 2 M HCl was added to stop the reaction; and absorbances at 450 nm and at 630 nm (as reference wavelength) were read, and A450nm-630nm values of the wells in the plates were recorded.

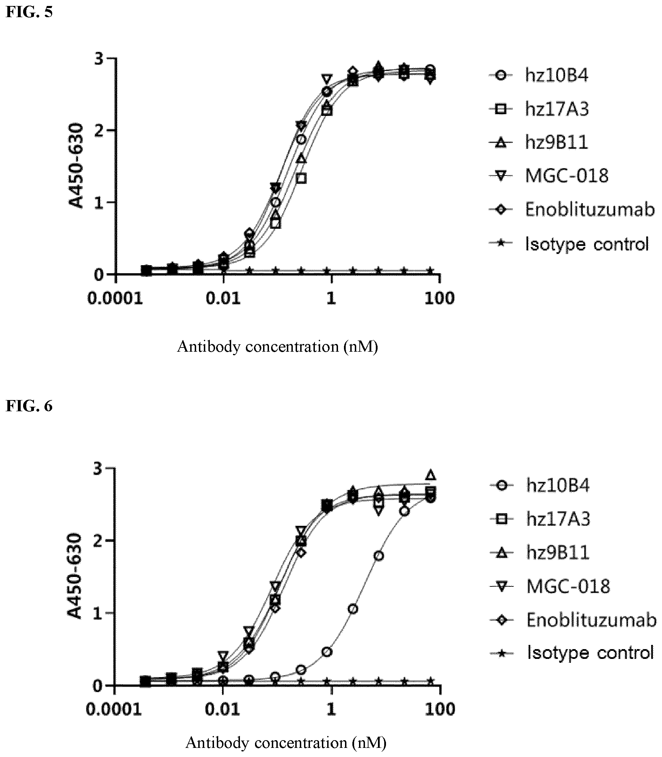

The experiment results showed that hz10B4, hz17A3, and hz9B11 as well as control antibody Enoblituzumab all could specifically bind to the recombinant human and cynomolgus B7H3, but had no binding activity to the recombinant mouse B7H3 (FIG. 5, FIG. 6, and FIG. 7). Respective half maximal effective binding concentration (EC50) values of the antibodies are shown in Table 9.

**TABLE 9. Detection of the binding of the anti-B7-H3 humanized antibodies to human, cynomolgus, and mouse B7-H3**

| EC50 (nM) | hz10B4 | hz17A3 | hz9B11 | MGC-018 | Enoblituzumab |
|---|---|---|---|---|---|
| Human B7-H3 | 0.1613 | 0.2805 | 0.2187 | 0.1168 | 0.1169 |
| Cynomolgus B7-H3 | 4.058 | 0.1083 | 0.1183 | 0.07819 | 0.1335 |

### Example 11: Analysis of affinities of anti-B7-H3 humanized antibodies

Affinities of the antibodies were determined by an assay including capturing Fc fragments of the antibodies with anti-human IgG Fc capture (AHC) biosensors using Octet QKe system instrument from Fortebio. For the assay, each of the antibodies (hz10B4, hz17A3, and hz9B11 as well as control antibodies MGC-018 and Enoblituzumab) was diluted to 4 µg/ml in PBS, and was allowed to flow through the surface of an ARC biosensor (Cat.: 18-0015, PALL) for 120 s. Recombinant human B7-H3-his extracellular domain protein (Accession No.: UniProtKB-Q5ZPR3, 1 aa-461 aa) was used as a mobile phase. The association time was 300 s and the dissociation time was 300 s. When the assay was finished, data from which the response values of blank control had been deducted were fitted to a 1:1 Langmuir binding model using software, and then kinetic constants for antigen-antibody binding were calculated.

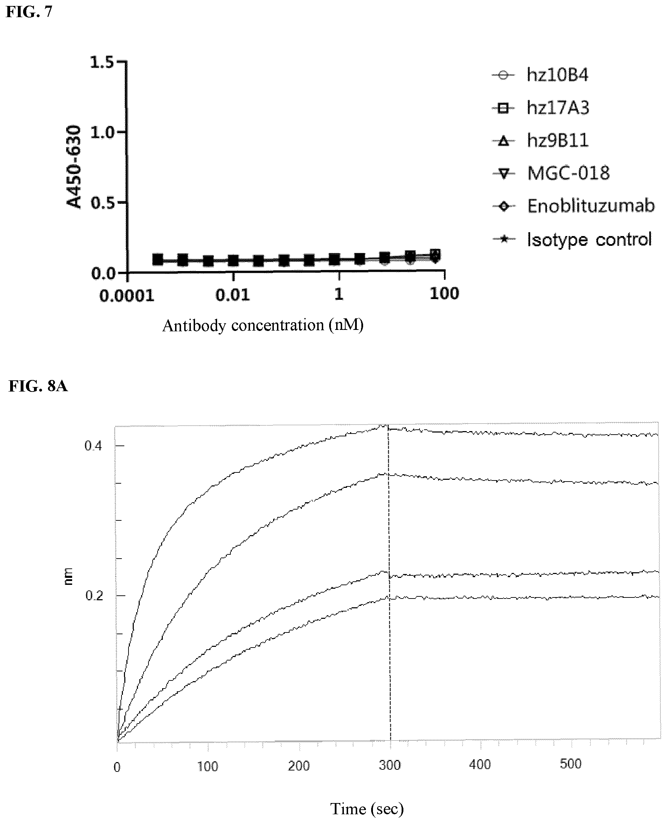

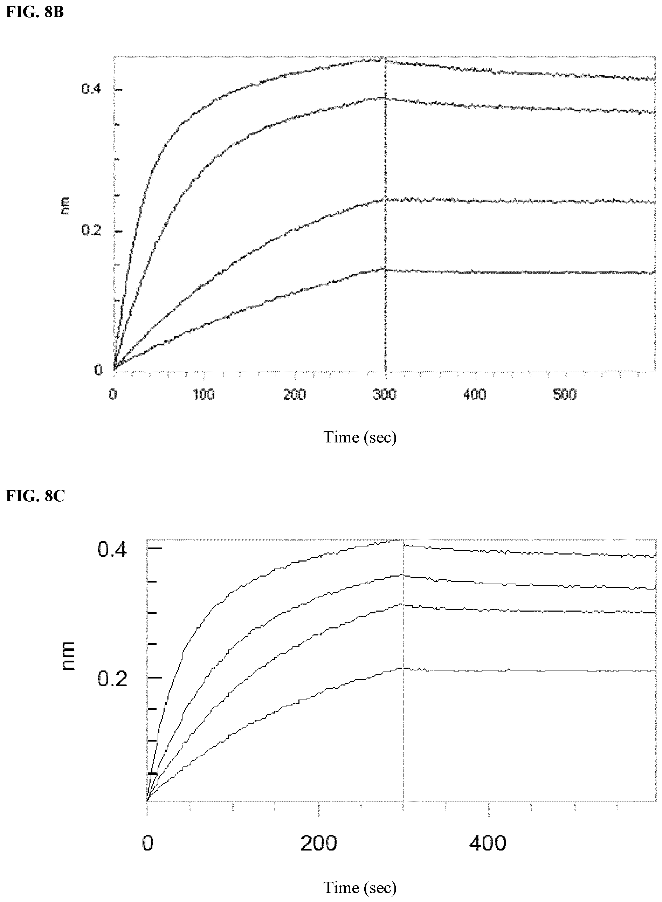

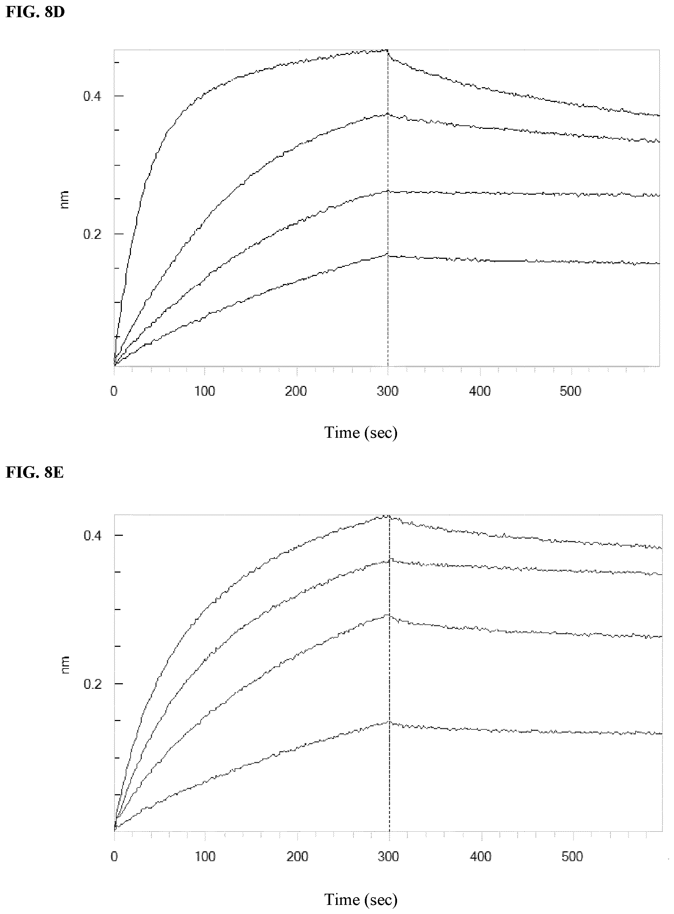

The reaction curves of hz10B4, hz17A3, hz9B11 and control antibodies MGC-018, Enoblituzumab with recombinant human B7-H3 protein are shown in FIG. 8. The curves were fitted and the affinities were calculated: the affinity (KD) of hz10B4 was 1.42E-10 M, the affinity (KD) of hz17A3 was 1.77E-10 M, the affinity (KD) of hz9B11 was 2.19E-10 M, the affinity (KD) of MGC-018 was 5.97E-10 M, and the affinity (KD) of Enoblituzumab was 9.50E-10 M. The detailed kinetic parameters are shown in Table 10 below. The results showed that hz10B4, hz17A3 and hz9B11 had high affinities for human B7-H3, superior to those of the control antibodies.

**TABLE 10. Detection results of the affinities of the anti-B7-H3 humanized antibodies for recombinant human B7-H3 extracellular domain protein**

| | KD value (M) | kon(1/Ms) | kdis(1/s) |
|---|---|---|---|
| hz10B4 | 1.42E-10 | 5.23E+05 | 7.44E-05 |
| hz17A3 | 1.77E-10 | 8.13E+05 | 1.44E-04 |
| hz9B11 | 2.19E-10 | 7.03E+05 | 1.54E-04 |
| MGC-018 | 5.97E-10 | 8.65E+05 | 5.17E-04 |
| Enoblituzumab | 9.50E-10 | 2.91E+05 | 2.76E-04 |

### Example 12: Binding of anti-B7-H3 humanized antibodies to cells naturally expressing human B7-H3 detected by FACS

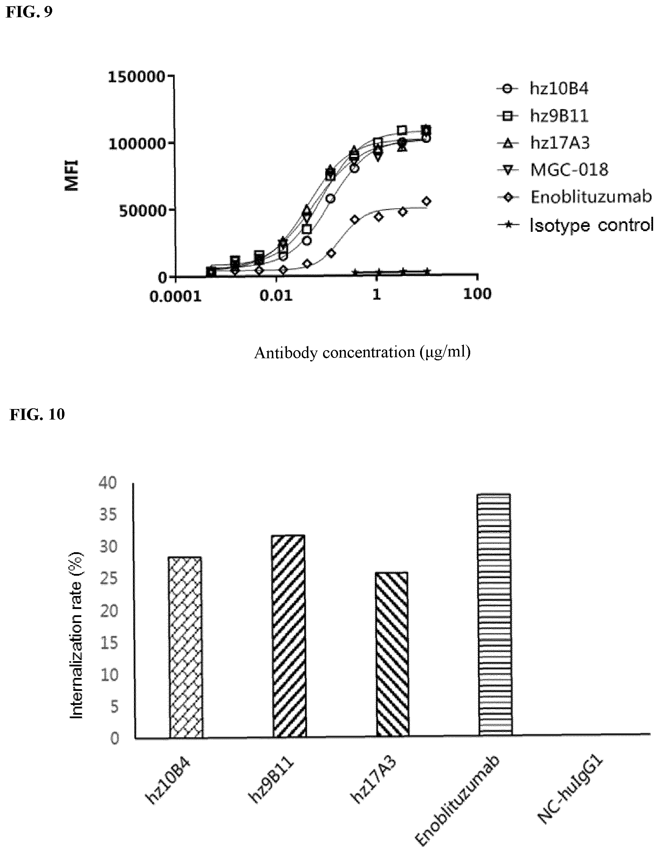

Suspensions of human ovarian carcinoma cells (SKVO3 cells) naturally expressing human B7-H3 were incubated with the humanized antibodies (hz9B11, hz10B4, and hz17A3; diluted in a 3-fold gradient from a concentration of 10 µg/ml, 10 concentrations in total) at 37 °C for 30 min. Controls as follows were set: (1) Positive Controls (PCs): control antibodies Enoblituzumab and MGC-018; (2) Negative Control (NC): an isotype control antibody. The cells were washed 3 times with PBS, and 1:200 diluted goat anti-human IgG-FITC (Cat.: F9512, Sigma) was added into the cells which then were incubated for 30 min. Then the cells were washed 3 times with PBS again, and the Mean Fluorescence Intensity (MFI) of the cells was measured by a flow cytometer (model B49007AD, SNAW31211, BECKMAN COULTER) to detect the binding abilities of the humanized antibodies to the B7-H3 protein on the surface of the SKVO3 cells. As detected by FACS, the humanized antibodies hz9B11, hz10B4, and hz17A3 all could bind to the B7-H3 protein on the surface of the SKVO3 cells, with binding capacities superior to that of the control antibody Enoblituzumab. Respective half maximal effective binding concentration (EC50) values of the antibodies are shown in Table 11, and the binding is shown in FIG. 9.

**TABLE 11. EC50 values of the binding of the anti-human B7-H3 humanized antibodies to SKVO3 cells detected by FACS**

| | EC50 (µg/ml) | | | | |
|---|---|---|---|---|---|
| | hz10B4 | hz9B11 | hz17A3 | MGC-018 | Enoblituzumab |
| SKVO3 | 0.1183 | 0.08277 | 0.04407 | 0.05649 | 0.1906 |

### Example 13: Internalization activities of anti-human B7-H3 humanized antibodies binding to B7-H3 on the surface of cells

MBA-MB-468 human breast cancer cells naturally expressing human B7-H3 were inoculated at a density of 3×10³ cells/well into 96-well cell culture plates and cultured for 24 hours. The cells were washed once with PBS and the supernatants were discarded. Humanized antibodies hz9B11, hz10B4, and hz17A3 as well as the positive control Enoblituzumab and a negative control (an isotype control antibody) were diluted to 10 µg/ml with RPMI 1640 (containing 10% FBS), and added to the MBA-MB-468 cells. Then the plates were divided into two groups: one group was placed in an electric heating thermostatic incubator at 37 °C, and one group was placed in a refrigerator at 4 °C as negative control. The plates as negative control were incubated for 1 hour, washed with PBS 3 times, and 1:200 diluted goat anti-human IgG Fc-FITC secondary antibody was added into the cells which then were incubated at 4 °C for 30 minutes. Then the cells were washed 3 times with PBS again, and the Mean Fluorescence Intensity (MFI) of the cells was measured by FACS. The plates of experiment groups were incubated at 37 °C for 5 h, and 1:200 diluted goat anti-human IgG Fc-FITC secondary antibody was added into the cells which then were incubated at 4 °C for 30 minutes. Then the cells were washed 3 times with PBS again, and the Mean Fluorescence Intensity (MFI) of the cells was measured by FACS. Internalization efficiency of the antibodies was calculated according to the formula: internalization rate % =100- (MFI of the sample incubated at 37 °C × 100 / MFI of the control sample incubated at 4 °C).

The experiment results (FIG. 10) showed that, the humanized antibodies hz9B11, hz10B4, and hz17A3 as well as the control antibody Enoblituzumab all could be endocytosed through the mediation by B7-H3.

### Example 14: Detection of killing activities of anti-human B7-H3 antibody-drug conjugates in tumor cells

Suspensions of SKVO3 and A431 cells naturally expressing human B7-H3 were inoculated at a density of 1×10³ cells/well into 96-well cell culture plates which were then placed into an incubator at 37 °C, 5% CO₂ for culturing overnight. Different concentrations of anti-human B7-H3 antibody-drug conjugates which were prepared through conjugating MMAE and named hz9B11-M, hz10B4-M, hz17A3-M, as well as positive controls Enoblituzumab-M and MGC-018-M and an ADC control MW14-M prepared (3-fold diluted from 11.1 µg/ml, 6 concentrations in total, each in triplicate wells) were added into the cells which were then placed and cultured in an incubator at 37 °C, 5% CO₂. 4 days later, cell killing activities of the anti-B7-H3 antibody-drug conjugates were detected using Cell Counting Kit-8 (CCK-8 kit).

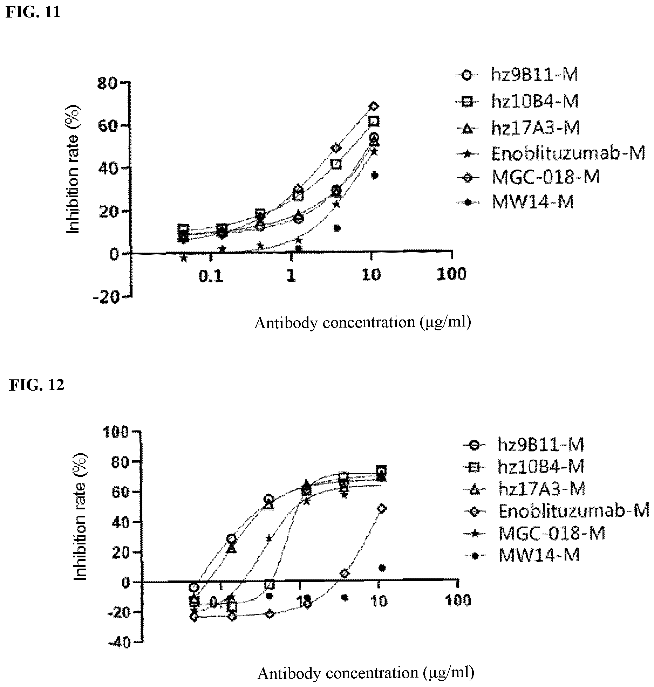

The results showed (FIG. 11 and FIG. 12) that, each anti-B7-H3 antibody-drug conjugate could kill tumor cells dose-dependently. On A431 cells, hz10B4-M had an activity slightly better than those of hz9B11-M and hz17A3-M, comparable to that of the control antibody-durg conjugate MGC-018-M, and obviously superior to that of Enobiluzumab-M. On SKOV-3 cells, hz9B11-M and hz17A3-M at low concentrations showed certain activities, which were obviously superior to those of the control antibody-drug conjugates; and hz10B4-M showed an activity comparable to that of control antibody-drug conjugate MGC-018-M, and obviously superior to that of Enoblituzumab-M.

### Example 15: Pharmacodynamic evaluation of anti-B7-H3 antibody-drug conjugates in a subcutaneous transplantation tumor model of A431 cells in nude mice

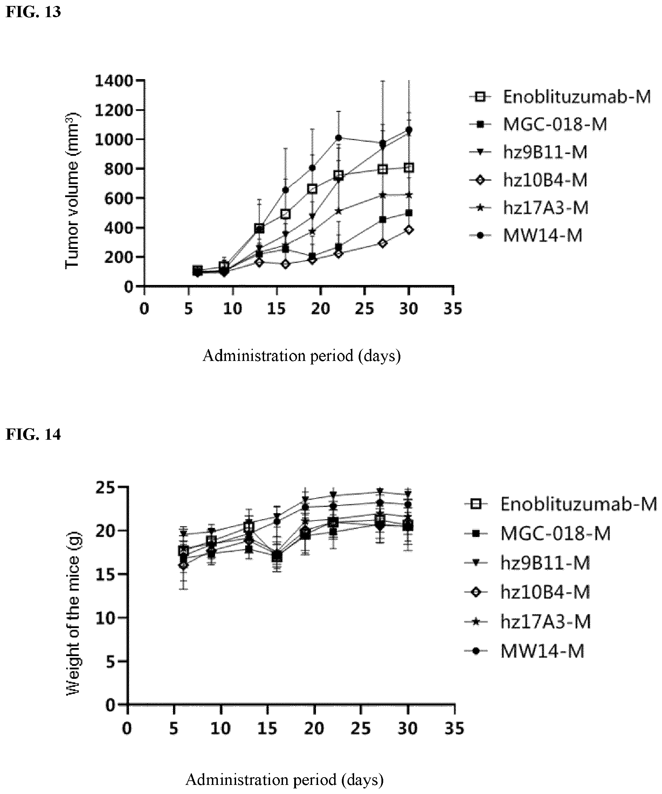

Five weeks old male BALB/c nude mice were subcutaneously inoculated with 3×10⁶ A431 cells, and randomly grouped with 5 mice per group when the tumors grew to around 100 mm³. Grouping as well as administration dosage and frequency for each group are shown in Table 15. The mice in each group were injected intravenously twice a week, 4 times in total, and at the same time, the tumor volume and body weight of each mouse were measured. After the administration was stopped, the mice were observed continuously for 2 weeks, and when a nude mouse lost weight more than 15% or when a nude mouse had a tumor volume exceeding 3000 mm³ or when a whole group of nude mice had an average tumor volume exceeding 2000 mm³, the experiment was stopped, and the mouse/mice was/were euthanized. Grouping as well as administration scheme of the nude mice are shown in Table 12, and tumor volume change curves and weight change curves of the nude mice are shown in FIG. 13 and FIG. 14 respectively.

**Table 12. Grouping as well as administration scheme of the nude mice**

| Group | Drug | Administration dosage | Administration frequency |
|---|---|---|---|
| 1 | hz10B4-M | 6. 25 mg/kg | Biw×4 |
| 2 | hz9B11-M | 6. 25 mg/kg | Biw×4 |
| 3 | hz17A3-M | 6. 25 mg/kg | Biw×4 |
| 4 | Enoblituzumab-M | 6. 25 mg/kg | Biw×4 |
| 5 | MGC-018-M | 6. 25 mg/kg | Biw×4 |
| 6 | ADC control MW14-M | 6. 25 mg/kg | Biw×4 |

As shown in FIGs. 13-14, in the subcutaneous transplantation tumor model of A431 cells in nude mice, hz10B4-M exhibited the best antitumor effect, which was basically equivalent to or slightly better than that of MGC-018-M; the antitumor effect of hz17A3-M took the second place, and superior to that of Enoblituzumab-M; and hz9B11-M showed a certain efficacy, but weaker than those of hz10B4-M and hz17A3-M. No obvious toxic effect of the small molecule MMAE used for preparing the ADCs was observed, and the body weight of animals in each experimental group steadily increased and there was no obvious difference in body weight between the experiment and control groups.

Although the specific embodiments of the invention have been described in detail, those skilled in the art will understand that according to all the teachings that have been disclosed, various modifications and substitutions can be made to those details, and these changes are all within the protection scope of the present invention. The full scope of the invention is given by the appended claims and any equivalents thereof.

In the description of this specification, the description referring to the terms such as "an embodiment", "some embodiments", "exemplary embodiments", "example", "specific example", "some examples" or the like means that the specific features, structures, materials or characteristics described with reference to the embodiment or example are included in at least one embodiment or example of the present disclosure. In this specification, exemplary descriptions of the foregoing terms do not necessarily refer to the same embodiment or example. In addition, the described specific features, structures, materials, or characteristics may be combined in a proper manner in any one or more of the embodiments or examples.

## Claims

1. An anti-human B7-H3 antibody or fragment thereof, **characterized in that** the anti-human B7-H3 antibody or fragment thereof comprises:
a heavy chain variable region (VH) comprising complementarity-determining regions (CDRs) 1, 2 and 3, wherein the VH CDR1 comprises an amino acid sequence having at least 75% identity to the amino acid sequence of a selected VH CDR1, the VH CDR2 comprises an amino acid sequence having at least 75% identity to the amino acid sequence of a selected VH CDR2, and the VH CDR3 comprises an amino acid sequence having at least 75% identity to the amino acid sequence of a selected VH CDR3;
a light chain variable region (VL) comprising CDRs 1, 2 and 3, wherein the VL CDR1 comprises an amino acid sequence having at least 75% identity to the amino acid sequence of a selected VL CDR1, the VL CDR2 comprises an amino acid sequence having at least 75% identity to the amino acid sequence of a selected VL CDR2, and the VL CDR3 comprises an amino acid sequence having at least 75% identity to the amino acid sequence of a selected VL CDR3;
wherein the amino acid sequences of the selected VH CDRs 1, 2 and 3 and the amino acid sequences of the selected VL CDRs 1, 2 and 3 are selected from the group consisting of:
the amino acid sequences of the selected VH CDRs 1, 2 and 3 are as shown in SEQ ID NOs: 40, 41 and 42 respectively, and the amino acid sequences of the selected VL CDRs 1, 2 and 3 are as shown in SEQ ID NOs: 43, 44 and 45 respectively;
the amino acid sequences of the selected VH CDRs 1, 2 and 3 are as shown in SEQ ID NOs: 46, 47 and 48 respectively, and the amino acid sequences of the selected VL CDRs 1, 2 and 3 are as shown in SEQ ID NOs: 49, 50 and 51 respectively; and
the amino acid sequences of the selected VH CDRs 1, 2 and 3 are as shown in SEQ ID NOs: 52, 53 and 54 respectively, and the amino acid sequences of the selected VL CDRs 1, 2 and 3 are as shown in SEQ ID NOs: 55, 56 and 57 respectively.

2. The anti-human B7-H3 antibody or fragment thereof according to claim 1, **characterized in that** the amino acid sequence having at least 75% identity to the selected VL CDR3 which comprises the amino acid sequence as shown in SEQ ID NO: 51 is QQWSX₁X₂PLT, wherein X₁ is S or A, and X₂ is N, A, S, or Q.

3. The anti-human B7-H3 antibody or fragment thereof according to claim 1, **characterized in that** the VH comprises an amino acid sequence having at least 75% identity to SEQ ID NO: 1, and the VL comprises an amino acid sequence having at least 75% identity to SEQ ID NO: 2.

4. The anti-human B7-H3 antibody or fragment thereof according to claim 1, **characterized in that** the VH comprises an amino acid sequence having at least 75% identity to SEQ ID NO: 3, and the VL comprises an amino acid sequence having at least 75% identity to SEQ ID NO: 4.

5. The anti-human B7-H3 antibody or fragment thereof according to claim 1, **characterized in that** the VH comprises an amino acid sequence having at least 75% identity to SEQ ID NO: 5, and the VL comprises an amino acid sequence having at least 75% identity to SEQ ID NO: 6.

6. The anti-human B7-H3 antibody or fragment thereof according to claim 3, **characterized in that** the VH comprises the amino acid sequence as shown in SEQ ID NO: 11 or 17, and the VL comprises the amino acid sequence as shown in SEQ ID NO: 12, 13, 14, 15, or 16.

7. The anti-human B7-H3 antibody or fragment thereof according to claim 4, **characterized in that** the VH comprises the amino acid sequence as shown in SEQ ID NO: 18, 21, 22, or 23, and the VL comprises the amino acid sequence as shown in SEQ ID NO: 19, 20, 58, 59, or 60.

8. The anti-human B7-H3 antibody or fragment thereof according to claim 5, **characterized in that** the VH comprises the amino acid sequence as shown in SEQ ID NO: 24, 34, 35, 36, or 37, and the VL comprises the amino acid sequence as shown in SEQ ID NO: 25, 26, 27, 28, 29, 30, 31, 32, or 33.

9. The anti-human B7-H3 antibody or fragment thereof according to claim 1, **characterized in that** the anti-human B7-H3 antibody or fragment thereof are capable of specifically binding to human B7-H3 extracellular domain, and has an affinity constant (KD) less than 5E-10 M for the human B7-H3 extracellular domain.

10. The anti-human B7-H3 antibody or fragment thereof according to claim 1, **characterized in that** the anti-human B7-H3 antibody or fragment thereof has cross reaction with monkey B7-H3, and has no binding activity to murine B7-H3.

11. The anti-human B7-H3 antibody or fragment thereof according to claim 1, **characterized in that** the anti-human B7-H3 antibody or fragment thereof has weak cross reaction with monkey B7-H3, and its binding activity to monkey B7-H3 is lower than its binding activity to human B7-H3.

12. The anti-human B7-H3 antibody or fragment thereof according to claim 1, **characterized in that** the anti-human B7-H3 antibody or fragment thereof are capable of specifically binding to B7-H3 on the cell surface and internalizing into the cell through the mediation by B7-H3.

13. A method for preparing an anti-human B7-H3 humanized antibody or fragment thereof, **characterized in that** the method comprises: on the basis of a non-human anti-B7-H3 antibody or fragment thereof, utilizing CDR grafting technology to achieve the humanization of heavy chain variable region and/or the humanization of light chain variable region, in order to obtain the anti-human B7-H3 antibody or fragment thereof according to any one of claims 1 to 12.

14. The method for preparing an anti-human B7-H3 humanized antibody or fragment thereof according to claim 13, **characterized in that** the step of utilizing CDR grafting technology to achieve the humanization of heavy chain variable region comprises:
(1) performing homology alignment using the heavy chain variable region sequence of a non-human anti-B7-H3 antibody or fragment thereof, and selecting a similar human antibody heavy chain template;
(2) grafting the VH CDRs 1, 2 and 3 of the non-human anti-B7-H3 antibody or fragment thereof into the human antibody heavy chain template;
(3) selecting a J region sequence according to the sequence of the VH CDR3, to achieve the humanization of heavy chain variable region and obtain the anti-human B7-H3 antibody or fragment thereof according to any one of claims 1 to 12.

15. The method for preparing an anti-human B7-H3 humanized antibody or fragment thereof according to claim 13, **characterized in that** the step of utilizing CDR grafting technology to achieve the humanization of light chain variable region comprises:
(1) performing homology alignment using the light chain variable region sequence of a non-human anti-B7-H3 antibody or fragment thereof, and selecting a similar human antibody light chain template;
(2) grafting the VL CDRs 1, 2 and 3 of the non-human anti-B7-H3 antibody or fragment thereof into the human antibody light chain template;
(3) selecting a JK region sequence according to the sequence of the VL CDR3, to achieve the humanization of light chain variable region and obtain the anti-human B7-H3 antibody or fragment thereof according to any one of claims 1 to 12.

16. The method for preparing an anti-human B7-H3 humanized antibody or fragment thereof according to any one of claims 13 to 15, **characterized in that** the method further comprises: after the CDR grafting, performing affinity maturation, site-directed mutagenesis in the CDR sequences and/or chemical modification of the humanized antibody.

17. A fusion molecule, **characterized in that** the fusion molecule comprises:
(1) A first active element, which is a first targeting moiety comprising the anti-human B7-H3 antibody or fragment thereof according to any one of claims 1 to 12;
(2) one or more second active elements, which comprise an effector element and/or a second targeting moiety;
wherein the first active element is linked to the second active element via a bond or a linker; and the one or more second active elements may be the same or different.

18. A polynucleotide, **characterized in that** the polynucleotide encodes the anti-human B7-H3 antibody or fragment thereof according to any one of claims 1 to 12 or the fusion molecule according to claim 17.

19. A construct, **characterized in that** the construct comprises the polynucleotide according to claim 18.

20. A host cell, **characterized in that** the host cell comprises the polynucleotide according to claim 18 or the nucleic acid construct according to claim 19.

21. A conjugate, **characterized in that** the conjugate comprises a first active element, which is a first targeting moiety comprising the anti-human B7-H3 antibody or fragment thereof according to any one of claims 1 to 12.

22. The conjugate according to claim 21, **characterized in that** the conjugate further comprises at least one second active element, wherein the first active element is covalently linked to the second active element.

23. The conjugate according to claim 22, **characterized in that** the second active element comprises one or more effector moieties.

24. The conjugate according to claim 23, **characterized in that** the effector moiety is a small molecule drug conjugate selected from the group consisting of one or more effector molecules or derivatives thereof in groups (1) to (3) as follows:
(1) small molecule compounds which are tubulin inhibitors: Maytansinoids, Monomethyl auristatin E, Monomethyl auristatin F, Monomethyl Dolastatin 10, Tubulysin and its derivatives, Cryptophycin and its derivatives, and Taltobulin;
(2) small molecule compounds which are topoisomerase inhibitors: PNU-159682 (the metabolite of Doxorubicin) and its derivatives, SN38 (the metabolite of Irinotecan) and its derivatives, and Exatecan;
(3) small molecule compounds which are DNA binding agents: PBD and its derivatives, and Duocarmycine and its derivatives.

25. A composition, comprising:
the anti-human B7-H3 antibody or fragment thereof according to any one of claims 1 to 12, the fusion molecule according to claim 17, the polynucleotide according to claim 18, the nucleic acid construct according to claim 19, the host cell according to claim 20, or the conjugate according to any one of claims 21 to 24; and
optionally pharmaceutically acceptable excipient(s).

26. A method for preparing an anti-human B7-H3 antibody or fragment thereof, comprising:
(1) culturing the host cell according to claim 20 under suitable conditions;
(2) separating and recovering an anti-human B7-H3 monoclonal antibody or fragment thereof, an anti-human B7-H3 chimeric antibody or fragment thereof, or an anti-human B7-H3 humanized antibody or fragment thereof.

27. Use of the anti-human B7-H3 antibody or fragment thereof according to any one of claims 1 to 12, the fusion molecule according to claim 17, the polynucleotide according to claim 18, the nucleic acid construct according to claim 19, the host cell according to claim 20, the conjugate according to any one of claims 21 to 24, and the composition according to claim 25 in the preparation of an agent for diagnosing a disease associated with B7-H3 overexpression.

28. Use of the anti-human B7-H3 antibody or fragment thereof according to any one of claims 1 to 12, the fusion molecule according to claim 17, the polynucleotide according to claim 18, the nucleic acid construct according to claim 19, the host cell according to claim 20, the conjugate according to any one of claims 21 to 24, and the composition according to claim 25 in the preparation of a medicament for treating a disease associated with B7-H3 overexpression.

29. The use according to claim 27 or 28, **characterized in that** the disease associated with B7-H3 overexpression is a solid tumor cancer.

30. The use according to claim 29, **characterized in that** the solid tumor cancer comprises: gastric cancer, colorectal cancer, liver cancer, pancreatic cancer, esophageal cancer, non-small cell carcinoma, prostate cancer, ovarian cancer, neuroblastoma, rhabdomyosarcoma, osteosarcoma, ewing sarcoma, wilms' tumor, and desmoplastic small round cell tumor.

31. A method for treating a subject having a cancer, comprising administering to the subject a therapeutically effective amount of a composition, which is the composition according to claim 25.

32. The method according to claim 31, **characterized in that** the cancer is a solid tumor cancer.

33. The method according to claim 32, **characterized in that** the solid tumor cancer comprises: gastric cancer, colorectal cancer, liver cancer, pancreatic cancer, esophageal cancer, non-small cell carcinoma, prostate cancer, ovarian cancer, neuroblastoma, rhabdomyosarcoma, osteosarcoma, ewing sarcoma, wilms' tumor, and desmoplastic small round cell tumor.
